# EUROPÄISCHE PATENTANMELDUNG

(11) **EP 4 749 284 A1**
(43) Veröffentlichungstag der Anmeldung: **27.05.2026**
(21) Anmeldenummer: 24215576.0
(22) Anmeldetag: 26.11.2024
(51) Int. Cl.: G01N 33/543, C07K 7/06, G01N 33/58, G01N 33/78

(54) **BESTIMMUNG EINES FREIEN THYROID-HORMONS**

(71) Anmelder: pes Diagnosesysteme GmbH, 04416 Markkleeberg (DE)
(72) Erfinder: FELLNER, Stephan, 04299 Leipzig (DE); ROCKTÄSCHEL, Jörg, 04289 Leipzig (DE)
(74) Vertreter: Mathys & Squire

(57) **Zusammenfassung**

Die vorliegende Erfindung betrifft ein Verfahren zur Bestimmung eines freien Thyroid-Hormons umfassend das Kombinieren einer Blutprobe mit einem Fängerreagenz, das ein Magnetpartikel-Konjugat mit einem Magnetpartikel und einem spezifischen Binder des zu bestimmenden Thyroid-Hormons umfasst; das Zugeben eines Detektorreagenzes, das einen Thyronin-Derivat-Tracer der Formel (II) umfasst; sowie das Auslösen und Quantifizieren einer Chemilumineszenz. Weiter betrifft die Erfindung einen Thyronin-Derivat-Tracer der Formel (II) und ein Kit für einen heterogenen Immunoassay zur Bestimmung eines freien Thyroid-Hormons umfassend ein Fänger- und ein Detektorreagenz.

## Beschreibung

### Gebiet der Erfindung

Die vorliegende Erfindung betrifft ein Verfahren zur Bestimmung eines freien Thyroid-Hormons umfassend das Kombinieren einer Blutprobe mit einem Fängerreagenz, das ein Magnetpartikel-Konjugat mit einem Magnetpartikel und einem spezifischen Binder des zu bestimmenden Thyroid-Hormons umfasst; das Zugeben eines Detektorreagenzes, das einen Thyronin-Derivat-Tracer der Formel (II) umfasst; sowie das Auslösen und Quantifizieren einer Chemilumineszenz. Weiter betrifft die Erfindung einen Thyronin-Derivat-Tracer der Formel (II) und ein Kit für einen heterogenen Immunoassay zur Bestimmung eines freien Thyroid-Hormons umfassend ein Fänger- und ein Detektorreagenz.

### Hintergrund der Erfindung

Schilddrüsenerkrankungen sind insbesondere in den Industrieländern weit verbreitet und in den USA sind beispielsweise 10 % der Bevölkerung hiervon betroffen. Zur Diagnose einer Schilddrüsenerkrankung wird in der klinischen Diagnostik primär ein Test auf das Thyroid-stimulierende-Hormon (TSH) durchgeführt und in vielen Fällen zusätzlich oder nachträglich ein oder beide Schilddrüsenhormon-Parameter freies Thyroxin (fT4) und freies Triiodthyronin (fT3) bzw. alternativ hierzu Gesamt-Triiodthyronin (TT3) bestimmt.

Die Schilddrüsenhormone Triiodthyronin (T3), Thyroxin (T4) und ihr hormonell inaktiver Metabolit reverses Triiodthyronin (rT3) sind schlecht wasserlöslich und sind für den Transport im Blutstrom stark Protein-gebunden.

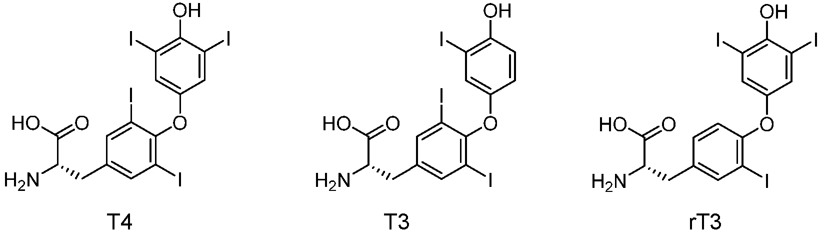

Die drei Proteine Thyroxin-bindendes Globulin (TBG), Präalbumin (Transthyretin) und Albumin, zusammenfassend auch T4-bindende Proteine genannt, übernehmen hierbei die reversible Bindung von T3 und T4 mit in genannter Proteinreihenfolge sinkendem Anteil.

Durch die beschriebene Protein-Bindung liegt T4 nur zu etwa 0,03 % in freier Form und T3 nur zu etwa 0,3 % in freier Form im Blut vor.

Da nur der frei vorliegende Anteil der Schilddrüsenhormone in die Zellen vordringen kann, um dort seine hormonelle Aktivität zu entfalten, wird generell die freie Konzentration eines Schilddrüsenhormons als klinisch aussagekräftiger angesehen, als dessen Gesamtkonzentration (also die Summe des proteingebundenen Schilddrüsenhormons und des freien Schilddrüsenhormons) im Blut (Thienpont et al., Best Practice & Research Clinical Endocrinology & Metabolism 27 (2013) 689-700; Spencer, Thyroid 33(4) (2023) 407-419).

Dementsprechend wird fT4 als Laborparameter der Bestimmung von Gesamt-T4 (TT4) vorgezogen, obwohl im Falle des fT4 die Analytenkonzentration um einen Faktor 3000 niedriger ist und die Bestimmung hier eine deutlich größere Herausforderung darstellt.

Im Falle von T3 wäre aus der eben beschriebenen theoretischen Sicht ebenfalls fT3 der bessere Laborparameter. Die fT3-Konzentration im Blut ist jedoch noch einmal um einen Faktor 3 niedriger als für fT4 und die bestehenden Assays für fT3 weisen eine vergleichsweise hohe Fehleranfälligkeit auf. Aufgrund dieses Sachverhaltes gibt es in der Literatur unterschiedliche Auffassungen darüber, ob derzeit fT3 oder Gesamt-T3 (TT3) als Laborparameter eingesetzt werden sollte (Van Uytfanghe et al., Thyroid 33(9) (2023) 1013-1028; Favresse et al., Endocrine Reviews 39(5) (2018) 830-850).

Dass sowohl für die bestehenden fT4- als auch die bestehenden fT3-lmmunoassays noch Verbesserungspotential insbesondere bezüglich der Verringerung der Fehleranfälligkeit dieser Assays besteht, ist in der Literatur gut dokumentiert. (Külz et al., Clinical Chemistry and Laboratory Medicine 60(6) (2022) 877-885; Westbye et al., Clinical Biochemistry 121-122 (2023) 110676; Favresse et al., Endocrine Reviews 39(5) (2018) 830-850; Thienpont et al., Best Practice & Research Clinical Endocrinology & Metabolism 27 (2013) 689-700). Die Laborparameter fT3, fT4, TT3 und TT4 werden in der klinischen Routinediagnostik fast ausschließlich mit Immunoassays auf vollautomatisierten Testplattformen bestimmt.

Es gibt auch die Möglichkeit diese Hormone mit LC-MS-Methoden nachzuweisen, wobei im Falle der freien Schilddrüsenhormone ein Dialyseschritt oder ein Ultrafiltrations-Schritt vorgeschaltet werden muss. Diese Methoden sind aber aufgrund des hohen technischen Aufwandes weit davon entfernt in der klinischen Routinediagnostik einsetzbar zu seien. Sie werden jedoch bereits als Referenzmethoden, z.B. für die Standardisierung der Routine-Immunoassaymethoden, eingesetzt. Das IFCC-Committee zur Standardisierung von Thyroid-Funktionstests (C-STFT) hat dazu ein Labornetzwerk etabliert, das diese Analysen für Referenzmessungen anbietet (Vesperet al., Clinica Chimica Acta 519 (2021) 183-186).

Für die in der Routineanalytik eingesetzten Immunoassays für die freien Schilddrüsenhormone werden mehrere mögliche Assayformate bzw. Testabläufe verwendet, die in der Literatur gut beschrieben sind (Christophides: Free Analyte Immunoassay, 123-137 from Wild ,The Immunoassay Handbook` 2013, fourth Edition, Elsevier; Spencer, Thyroid 33(4) (2023) 407-419). Grundsätzlich kann man hier zwischen 2-Step-Assays und 1-Step-Assays unterscheiden.

Beim 2-Step-Assay wird in einem ersten Schritt die Blutprobe mit einem ersten Reagenz vermischt, das auch den an eine feste Phase gebundenen Anti-Thyroid-Hormon-Antikörper enthält. Nach einer Inkubationszeit folgt bei diesem Assayformat ein Waschschritt, der die Mischung aus nicht-gebundenem Reagenz und Probe entfernt und nur den an die feste Phase gebundenen Antikörper und die Substanzen, die an ihn angebunden sind, zurücklässt. In einem zweiten Schritt wird ein Konjugat aus einem Thyroid-Hormon-Analog und einer Tracersubstanz zugegeben und wieder inkubiert. Nach erfolgter Inkubation wird erneut gewaschen, um ungebundenen Tracer zu entfernen, und der an die feste Phase gebundene Tracer quantifiziert.

1-Step-Assays beinhalten üblicherweise nur einen Waschschritt nach abgeschlossener Inkubation. Hier gibt es grundsätzlich zwei verschiedene Varianten die Assayreagenzien zu kombinieren: die Labeled-Analog-Tracer-Methode, bei der die Signal-erzeugende Komponente an das Thyroid-Hormon-Analog gebunden ist, und die Labeled-Antibody-Method, bei der die Signal-erzeugende Komponente an den Antikörper gebunden ist. Typischerweise wir das Reagenz, das das Thyroid-Hormon-Analog beinhaltet, beim 1-Step-Assay zeitverzögert zugegeben.

Alle drei genannten Assayformate haben ihre Vor- und Nachteile.

Typischerweise weisen die 1-Step-Methoden eine höhere Sensitivität und eine etwas höhere Präzision aufgrund der geringeren Anzahl an Assay-Prozessschritten, der tendenziell längeren Inkubationszeit der Assayreagenzien miteinander und bezogen auf die Labeled-Antibody Method eine homogenere Verteilung des Antikörpers in der Lösung auf. Der Nachteil des 1-Step-Assays besteht jedoch darin, dass während der Assay-Inkubation das Reagenz mit dem Thyroid-Hormon-Analog in Kontakt zur Probe und damit zu den darin enthaltenen T4-bindenden Proteinen kommt. Die T4-bindenden Proteine haben naturgemäß eine - je nach Reagenziendesign höhere oder niedrigere - Rest-Affinität zum Thyroid-Hormon-Analog des Assays und reduzieren hierdurch seine Aktivität. Dies ist deshalb kritisch, da Patientenproben sehr unterschiedliche Konzentrationen an T4-bindenden Proteinen enthalten können. Beispielsweise enthält das Plasma von schwangere Frauen im 3. Trimester der Schwangerschaft etwa die dreifache Konzentration des wichtigsten T4-bindenden Proteins TBG. Auch können erbliche Besonderheiten eines Patienten z.B. in der Sequenz des Albumins zu einer veränderten Affinität dieses Proteins zum Tracer in dieser speziellen Patientenprobe führen (z.B. familiäre dysalbuminämische Hyperthyroxinämie oderAnalbuminämie) oder alternativ das Analogkonjugat durch in der Probe vorhandene Autoantikörper in seiner Aktivität beeinflusst werden (Spencer, Thyroid 33(4) (2023) 407-419). Die Modulation der Tracer-Aktivität durch Proben mit Konzentrationen / Affinitäten der T4-bindenden Proteine, die vom typischen Wert abweichen, können im 1-Step-Assay unabhängig von der Analytenkonzentration zu einem veränderten Assaysignal und damit zu einer Verfälschung des Messwertes führen.

Der zuletzt genannte Fehlermechanismus ist im 2-Step Assay ausgeschlossen, da die Probe mit ihren T4-bindenden Proteinen nach der ersten Inkubation weggewaschen wird, bevor der Thyroid-Hormon-Analog-Tracer zugegeben wird. Generell werden so im 2-Step Assay Interferenzen durch die Interaktion von Probenbestandteilen mit dem Analog-Tracer wirkungsvoll unterbunden.

In der Patent-Literatur sind viele Ansätze zu finden, die für 1-Step-Assayformate die Interaktion zwischen Thyroid-Hormon-Analog-Reagenz und den T4-bindenden-Proteinen der Probe unterbinden sollen. Eine Hauptroute ist die sterische Hinderung des Thyroid-Hormon-Analogs durch Bindung an ein Protein, z.B. Albumin oder HRP, oder an die feste Phase des Assays. Nach Ansicht der Autoren führen diese Maßnahmen aber nur zu einer Reduktion der Intensität der Interaktion und nicht zu ihrer vollständigen Eliminierung.

Ein Problem der aktuell verwendeten Routine-Assays besteht darin, dass diese Assays Reagenzien verwenden, die nur mit sehr eingeschränkter Reproduzierbarkeit mit den gleichen Eigenschaften erneut hergestellt werden können. Das betrifft insbesondere die beiden aktiven Assaykomponenten, die an der Bildung des Immunkomplexes beteiligt sind. Den Binder und das Thyroid-Analog-Konjugat.

Auf Seite des Antikörpers bzw. Binders verwenden die Mehrzahl der eingesetzten klinischen Assays für die Bestimmung von freien Schilddrüsenhormonen polyklonale Antikörper. Polyklonale Antikörper haben z.B. im Vergleich zu monoklonalen Antikörpern den Nachteil, dass bei der Herstellung eines neuen Lots ein neues Tier immunisiert werden muss und das resultierende Produkt aufgrund der individuell anders verlaufenden Immunreaktion im neuen Organismus andere Eigenschaften haben wird.

Auf Seite des Thyroid-Analog-Konjugates sind kommerzielle Reagenzien oder Reagenzien-Vorstufen erhältlich, bei denen das Thyroid-Analog, meistens T3, kovalent an ein Protein, z.B. Albumin oder HRP, angebunden ist. Dieser Ansatz löst zwei Probleme auf einmal. Zum Ersten ist die Löslichkeit der Hormon-Analoga und des verwendeten Farbstoffes durch die Anbindung an das große Protein gewährleitet, zum Zweiten ist das Thyroid-Hormon durch die Anbindung an das Protein sterisch vor einer allzu starken Anbindung durch T4-bindende Proteine geschützt. Üblicherweise werden hier mehrere T3-Gruppen pro Protein nicht-selektiv an einen Teil der bestehenden funktionellen Gruppen, z.B. Lysine, des Proteins gekoppelt. Durch dieses Verfahren entsteht in einem Reagenzlot eine ganze Population von z.B. (T3)ₓ-Albumin-Molekülen, bei denen jedes einzelne Tracermolekül ein unterschiedliches molares Verhältnis von T3 zu Protein aufweist und für das die T3-Gruppen zudem an individuelle Lysin-Gruppen des Proteins gebunden sind. Da z.B. humanes Serum-Albumin (HSA) 59 Lysin-Gruppen aufweist, kann man sich vorstellen, dass es hier viele Möglichkeiten der Ankoppelung gibt. Zur Herstellung eines Tracers müssen zusätzlich an das resultierende T3-Albumin noch mehrere Farbstoffgruppen angekoppelt werden, so dass diese heterogene Vielfalt ebenso für die angekoppelten Farbstoffmoleküle entsteht. Für einen so hergestellten Tracer ist es nicht möglich, bei der Herstellung einer neuen Charge ein identisches Produkt herzustellen. In der klinischen Diagnostik ist es aber gerade wichtig, dass die Reagenzien bei jeder Chargenherstellung wieder die gleichen Eigenschaften aufweisen, da ansonsten kostspielige Revalidierungsprozeduren für jede Produktionscharge notwendig werden.

Unterbleibt eine solche Revalidierung bei nicht-reproduzierbar herstellbaren Neuchargen, ist mit unerwarteten Fehlern beim klinischen Einsatz zu rechnen.

Aufgrund der dargestellten Situation, ist die Notwendigkeit gegeben, die bestehenden fT4 und fT3-Assays weiter zu verbessern. Die Assays sollen kostengünstige und nur reproduzierbar herstellbare Aktiv-Komponenten (insbesondere Binder und Thyroid-Analog-Konjugate) einsetzen, eine geringe Anfälligkeit gegen Interferenzen aufweisen sowie eine hohe analytische und klinische Performance (z.B. Sensitivität, Genauigkeit, Reproduzierbarkeit, Stabilität) liefern.

### Kurzdarstellung der Erfindung

Die vorliegende Erfindung betrifft einen Thyronin-Derivat-Tracer der Formel (II):

CL-Y-TX (II).

CL steht für eine Chemilumineszenzgruppe.

Y steht für ein lineares Peptid mit 3 bis 11 Aminosäuren, wobei mindestens die Hälfte der Aminosäuren des linearen Peptids saure Aminosäuren sind.

TX steht für eine Gruppe der Formel (III) oder (IV):

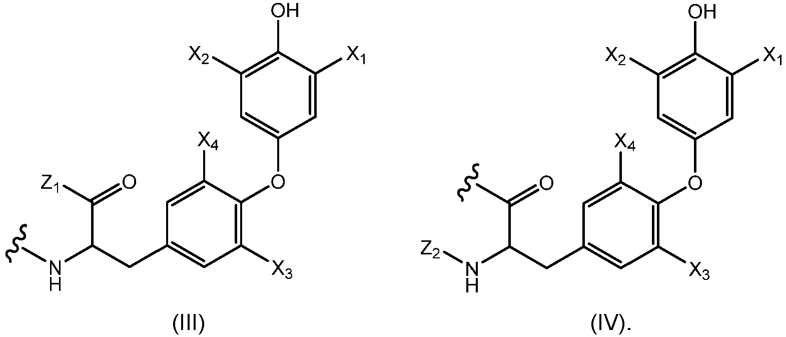

X₁, X₂, X₃ und X₄ stehen unabhängig voneinander für I, H, Br, CI oder CN.

Z₁ steht für OH oder NH₂, und Z₂ steht für H oder C(O)CH₃.

Die vorliegende Erfindung betrifft auch ein Verfahren zur quantitativen Bestimmung eines freien Thyroid-Hormons. Das Verfahren umfasst das Bereitstellen einer Blutprobe. Das Verfahren umfasst weiter das Kombinieren der Blutprobe mit einem Fängerreagenz in einem Reaktionsgefäß unter Erhalt eines ersten Gemisches, wobei das Fängerreagenz ein Magnetpartikel-Konjugat umfasst, und wobei das Magnetpartikel-Konjugat einen Magnetpartikel und einen spezifischen Binder des zu bestimmenden Thyroid-Hormons umfasst. Das Verfahren umfasst weiter das Aufarbeiten des ersten Gemisches. Das Verfahren umfasst weiter das Zugeben eines Detektorreagenzes in das Reaktionsgefäß unter Erhalt eines zweiten Gemisches, wobei das Detektorreagenz einen Thyronin-Derivat-Tracer der hierin beschriebenen Formel (II) umfasst. Das Verfahren umfasst weiter das Aufarbeiten des zweiten Gemisches. Das Verfahren umfasst weiter das Auslösen einer Chemilumineszenz durch Zugabe mindestens eines Vorbereitungsreagenzes und mindestens eines Initiationsreagenzes und das Quantifizieren der Chemilumineszenz.

Das Aufarbeiten des ersten Gemisches umfasst das Inkubieren des ersten Gemisches, das Immobilisieren des Magnetpartikel-Konjugates an der Wand des Reaktionsgefäßes, das Entfernen der flüssigen Phase und das Waschen der festen Phase durch mehrmalige Abgabe und Aufnahme eines Waschpuffers.

Das Aufarbeiten des zweiten Gemisches umfasst das Resuspendieren der Partikel des Magnetpartikel-Konjugat im Detektorreagenz, das Inkubieren des zweiten Gemisches, das Immobilisieren des Magnetpartikel-Konjugat an der Wand des Reaktionsgefäßes, das Entfernen der flüssigen Phase und das Waschen der festen Phase durch mehrmalige Abgabe und Aufnahme eines Waschpuffers.

Die vorliegende Erfindung betrifft auch ein Kit für einen heterogenen Immunoassay zur Bestimmung eines freien Thyroid-Hormons. Das Kit umfasst ein Fängerreagenz umfassend ein Magnetpartikel-Konjugat, wobei das Magnetpartikel-Konjugat einen Magnetpartikel und einen spezifischen Binder des entsprechenden Thyroid-Hormones umfasst und ein Detektorreagenz umfassend einen Thyronin-Derivat-Tracer der hierin beschriebenen Formel (II).

### Detaillierte Beschreibung der Erfindung

Unter "Thyroid-Hormone" oder "Schilddrüsenhormone" werden im Rahmen der vorliegenden Erfindung die in den Follikelepithelzellen der Schilddrüse (Thyreozyten) gebildeten jodhaltigen Hormone 3,3`,5-Triiod-L-thyronin (Triiodthyronin, T3) und L-Thyroxin (Thyroxin, T4) verstanden. Die Begriffe "Thyroid-Hormone" und "Schilddrüsenhormone" werden im Rahmen der vorliegenden Erfindung analog verwendet. T3 und T4 liegen im Blut in hohem Maße proteingebunden vor. Ihr frei vorliegender Anteil (nicht proteingebundener Anteil) im Blut wird jeweils als freies Triiodthyronin (fT3) bzw. freies Thyroxin (fT4) bezeichnet.

Unter "Aminosäuren" werden im Rahmen der vorliegenden Erfindung α-Aminosäuren verstanden, also organische Verbindungen mit einer Aminogruppe, die am Cα-Atom unmittelbar benachbart zu einer endständigen Carboxygruppe steht. Bevorzugte "Aminosäuren" im Rahmen der vorliegenden Erfindung sind proteinogene Aminosäuren, besonders bevorzugt kanonische Aminosäuren. Unter "saure Aminosäuren" werden Aminosäuren verstanden, die mehr Carboxylgruppen als Aminogruppen aufweisen. Bevorzugte "saure Aminosäuren" im Rahmen der vorliegenden Erfindungen sind die proteinogenen Aminosäuren Asparaginsäure (D) und Glutaminsäure (E). Unter "basischen Aminosäuren" werden Aminosäuren verstanden, die mehr basische Gruppen als Carboxylgruppen aufweisen. Bevorzugte "basische Aminosäuren" im Rahmen der vorliegenden Erfindungen sind die proteinogenen Aminosäuren Lysin (K), Arginin (R).

Unter dem Begriff "Peptid" versteht man eine chemische Verbindung, die aus einer Verknüpfung mehrerer Aminosäuren hervorgegangen ist. Die einzelnen Aminosäuren eines Peptides sind dabei über Peptidbindungen zwischen ihren α-ständigen NH₂-Gruppen und α-ständigen Carboxy-Gruppen miteinander verknüpft. Verwendet man die gängigen "Einbuchstaben-Symbole" für Aminosäuren, so steht ganz links die N-terminale Aminosäure, d.h. die Aminosäure weist eine freie α-ständige NH₂-Gruppe auf. Diese Aminosäure ist mit ihrer α-ständigen Carboxy-Gruppe über eine Peptid-Bindung mit der α-Aminogruppe der folgenden Aminosäure kondensiert und so fort. Die letzte angebundene Aminosäure dieser Kette, die dann eine freie α-Carboxygruppe aufweist, wird C-terminale Aminosäure genannt. Als Beispiel sei das Peptid EEEEE angeführt, das aus 5 kondensierten Glutaminsäuren besteht und die folgende chemische Strukturformel besitzt:

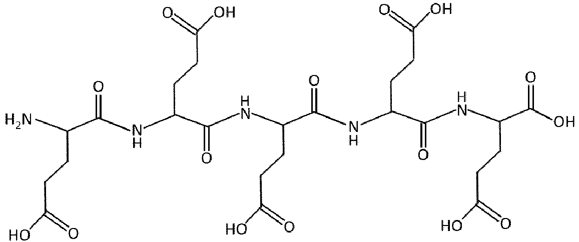

Unter dem Begriff "Tracer-Kurzformel" versteht man im Rahmen der vorliegenden Erfindung die Codierung der chemischen Struktur der erfindungsgemäßen Tracer CL-Y-TX (II), wie im Folgenden beschrieben. Die Tracer-Kurzformel beginnt mit dem Abkürzungsnamen der verwendeten Chemilumineszenzgruppe, z.B. "NSPSA", die ebenfalls in der detaillierten Beschreibung definiert ist. Die Aminosäuresequenz des Peptides Y ist in der Tracer-Kurzformel über die Einbuchstaben-Symbole der enthaltenen Aminosäuren beschrieben, deren Aufbau und Verknüpfung unter dem Begriff "Peptid" erklärt sind. Die Bindung zwischen Chemilumineszenzgruppe CL und Peptid Y sowie die Bindung zwischen Thyronin-Derivat TX und Peptid Y sind Peptidbindungen.

Dabei ist es grundsätzlich möglich, dass die Chemilumineszenzgruppe über eine Carboxygruppe an eine Amino-Gruppe des Peptids Y (bevorzugt der N-terminalen Aminosäure des Peptids Y) gebunden ist. Entsprechend ist es grundsätzlich möglich, dass das Thyronin-Derivat TX über eine Carboxygruppe an eine Amino-Gruppe des Peptids Y (bevorzugt der N-terminalen Aminosäure des Peptids Y) gebunden ist oder dass das Thyronin-Derivat TX über eine Aminogruppe an eine Carboxy-Gruppe des linearen Peptids Y (bevorzugt der C-terminalen Aminosäure des Peptids Y) gebunden ist.

Bevorzugt ist die Chemilumineszenzgruppe über eine Carboxygruppe mittels Peptidbindung an die α-Amino-Gruppe der N-terminalen Aminosäure des Peptides Y gebunden. Entsprechend ist die C-terminale Aminosäure des linearen Peptides Y bevorzugt mit dem Thyronin-Derivat TX über eine Peptidbindung verbunden.

Die genaue Struktur von TX wird in der Tracer-Kurzformel durch die Angabe der verwendeten Formelgruppe (beispielhaft (Illa) bis (IIIe) bzw. (IVa) bis (IVe)) festgelegt, die in der detaillierten Beschreibung der Erfindung definiert sind. Die TX-Strukturen vom Typ (III) sind mit einer Z₁-Gruppe, die TX-Strukturen vom Typ (IV) mit einer Z₂-Gruppe substituiert.

TX-Gruppen vom Typ (III) sind bevorzugt über eine Peptidbindung mit der α-Carboxy-Gruppe der C-terminalen Aminosäure des Peptides Y verbunden. TX-Gruppen vom Typ (IV) sind bevorzugt über eine Peptidbindung mit einer NH₂-Gruppe der Seitenkette der C-terminalen Aminosäure (für Lysin die ε-NH₂-Gruppe) des linearen Peptids Y verbunden.

Im Rahmen der vorliegenden Erfindung werden in den Tracer-Kurzformeln die Z₁- bzw. Z₂-Gruppen ebenfalls angegeben. So steht beispielsweise (IIIc)OH für eine TX-Gruppe der Formel (lllc), wobei Z₁ für OH steht.

Zur Verdeutlichung sind beispielhaft für zwei Tracer-Kurzformeln die Strukturformeln dargestellt:
- Strukturformel zur Tracer-Kurzformel NSPSA-EEEEEEE-(lllc)OH: 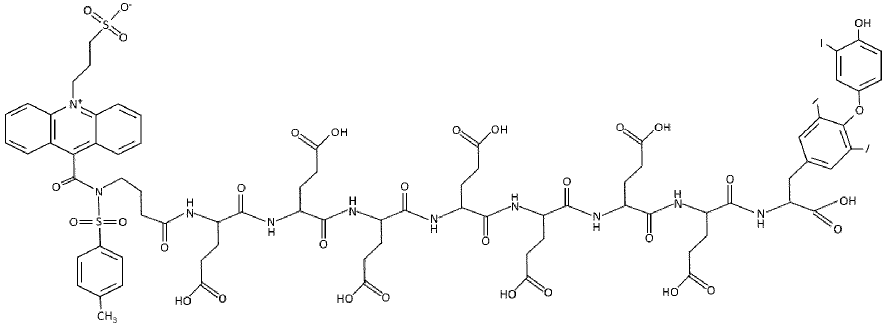
- Strukturformel zur Tracer-Kurzformel NSPSA-EKEK-(IVa)C(O)CH₃:

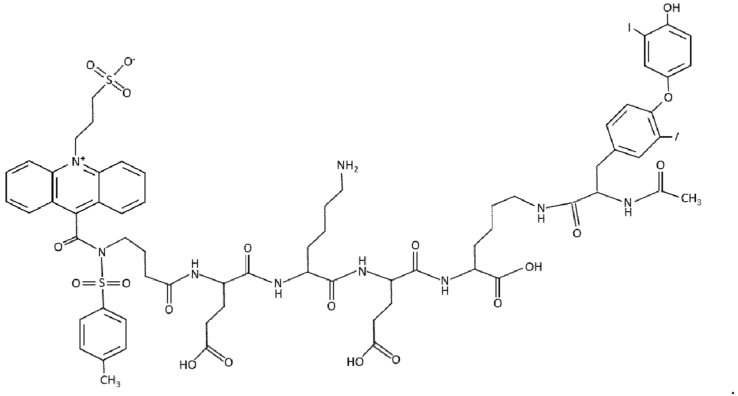

Unter dem Begriff "die Hälfte" wird einer von zwei gleichen Teilen eines Ganzen verstanden. Bei einem absoluten Wert erhält man die Hälfte durch Division des Wertes durch 2. Entsprechend bedeutet "mindestens die Hälfte" eines absolute Wertes die Hälfte dieses Wertes oder mehr. Im Falle einer ungeraden ganzen Zahl wird im Rahmen der vorliegenden Erfindung aufgerundet. Zum Beispiel ist bei einem Wert x = 8 "mindestens die Hälfte" 4 oder mehr. Zum Beispiel ist bei einem Wert x = 9 "mindestens die Hälfte" 5 oder mehr.

Unter dem Begriff "spezifische Binder" werden gemäß der vorliegenden Erfindung Verbindungen mit einer hohen Bindungsstärke zu dem zu bestimmenden Thyroid-Hormon, also entweder T3 oder T4, verstanden. Unter den Begriff "spezifische Binder" fallen im Rahmen der vorliegenden Erfindung sowohl Antikörper, als auch Antikörperfragmente (wie z.B. F(ab')₂ oder Fab-Fragmente), Nanobodies, Rezeptoren und Aptamere, sofern sie eine hohe Bindungsstärke zu dem zu bestimmenden Thyroid-Hormon aufweisen.

Ein Thyroid-Assay im Sinne der vorliegenden Erfindung weist nur eines der beiden Hormone, entweder T3 oder T4, spezifisch nach. Daher soll, z.B. im Falle eines fT4-Assays der Binder eine hohe Bindungsstärke zu T4 aufweisen, aber eine deutlich geringere Bindungsstärke (z.B. mindestens 2mal schwächer, bevorzugt mindestens 5mal schwächer, weiter bevorzugt mindestens 20mal schwächer) zu T3. Entsprechend soll im Falle eines fT3-Assays der Binder eine hohe Bindungsstärke zu T3 aufweisen, aber eine deutlich geringere Bindungsstärke (z.B. mindestens 2mal schwächer, bevorzugt mindestens 5mal schwächer, weiter bevorzugt mindestens 20mal schwächer) zu T4.

Unter dem Begriff "Tracer" werden im Rahmen der vorliegenden Erfindung aktive Verbindungen des Immunoassays verstanden, die eine Komponente, z.B. einen Farbstoff, enthalten, der im Immunoassays das Signal erzeugt. Im Rahmen der vorliegenden Erfindung ist dieser Farbstoff ein Chemilumineszenzfarbstoff.

Unter "Chemilumineszenzfarbstoff' wird im Rahmen dieser Erfindung eine chemische Verbindung bezeichnet, die durch Zugabe weiterer Chemikalien zur Chemilumineszenz angeregt werden kann, beispielsweise Verbindungen aus der Gruppe der Acridiniumester und Acridiniumsulfonamide.

Gemäß der vorliegenden Erfindung wird unter einer "hohen Bindungsstärke" eine Bindung mit einer Affinitäts-Konstante von mindestens 1*10⁹ I/mol, bevorzugt mindestens 1*10¹⁰ I/mol, weiter bevorzugt mindestens 2*10¹⁰ I/mol verstanden. Die Affinitätskonstante kann z.B. mittels Scatchard-Plot oder Surface-Plasmon-Resonace (z.B. mit einem Biacore-Gerät) bestimmt werden.

Unter dem Begriff "Größe" in Bezug auf Partikel wird jeweils der mittlere Durchmesser der verwendeten Partikel verstanden, wie er z.B. durch Elektronenmikroskopie gemessen werden kann.

"Annähernd" bedeutet im Rahmen der vorliegenden Erfindung eine Abweichung von weniger als 20 %, z.B. weniger als 10 % oder weniger als 5 %, besonders bevorzugt weniger als 2 %. "Annähernd sphärisch" bedeutet im Rahmen der vorliegenden Erfindung bezogen auf die Form von Partikeln, dass der größte Durchmesser und der kleinste Durchmesser eines Partikels nicht mehr als 20 % (z.B. weniger als 10 % oder weniger als 5 %, besonders bevorzugt weniger als 2 %) voneinander abweichen.

"Quantitativ" oder "quantitative Bestimmung" beschreibt die Bestimmung der Menge oder Konzentration einer Komponente, z.B. eines Thyroid-Hormons, in einer Probe.

Die vorliegende Erfindung betrifft einen Thyronin-Derivat-Tracer der Formel (II):

CL-Y-TX (II).

CL steht für eine Chemilumineszenzgruppe.

Y steht für ein lineares Peptid mit 3 bis 11 Aminosäuren, wobei mindestens die Hälfte der Aminosäuren des linearen Peptids saure Aminosäuren sind.

TX steht für eine Gruppe der Formel (III) oder (IV):

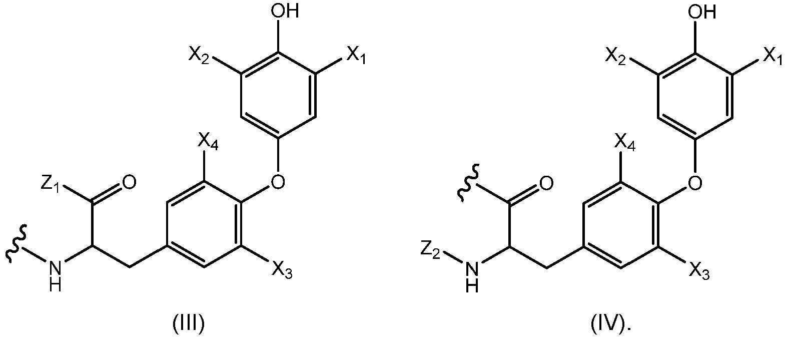

X₁, X₂, X₃ und X₄ stehen unabhängig voneinander für I, H, Br, CI oder CN.

Z₁ steht für OH oder NH₂, und Z₂ steht für H oder C(O)CH₃.

In Formel (II) steht Y für ein lineares Peptid mit 3 bis 12 Aminosäuren. Das lineare Peptid umfasst mindestens 3, zum Beispiel mindestens 4 oder mindestens 5 oder mindestens 6 Aminosäuren. Das lineare Peptid umfasst nicht mehr als 12, zum Beispiel nicht mehr als 11 oder nicht mehr als 10 oder nicht mehr als 9 oder nicht mehr als 8 Aminosäuren. Bevorzugt umfasst das lineare Peptid 4 bis 10 Aminosäure, besonders bevorzugt 5 bis 8 Aminosäuren.

Als Aminosäuren sind im Rahmen der vorliegenden Erfindung L- Aminosäuren und D-Aminosäuren geeignet. Zum Beispiel können die Aminosäuren des linearen Peptids D-Aminosäuren sein, oder die Aminosäuren des linearen Peptids können L-Aminosäuren sein, oder das lineare Peptid kann sowohl D- als auch L-Aminosäuren enthalten. Bevorzugt sind die Aminosäuren des linearen Peptids D-Aminosäuren. Zum Beispiel kann zumindest eine Aminosäure des linearen Peptids eine D-Aminosäure sein. Es können 2 oder 3 oder mindestens die Hälfte der Aminosäuren des linearen Peptids D-Aminosäuren sein.

Mindestens die Hälfte der Aminosäuren des linearen Peptids sind saure Aminosäuren. Bevorzugt sind mindestens die Hälfte der Aminosäuren des linearen Peptids Glutaminsäuren oder Asparaginsäuren. Zum Beispiel können mehr als die Hälfte der Aminosäuren des linearen Peptids Glutaminsäuren oder Asparaginsäuren oder alle Aminosäuren des linearen Peptids Glutaminsäuren oder Asparaginsäuren sein.

Es kann ebenfalls bevorzugt sein, dass das lineare Peptid aus sauren und basischen Aminosäuren gebildet wird. Bevorzugt ist als basische Aminosäure Lysin enthalten.

Das lineare Peptid kann 1 bis 6 Lysine, z.B. 1-5 oder 1-4 oder 1-3 Lysine umfassen. Das lineare Peptid kann zum Beispiel mindestens 2 oder mindestens 3 Lysine umfassen. Das lineare Peptid kann zum Beispiel nicht mehr als 5 oder nicht mehr als 4 oder nicht mehr als 3 Lysine umfassen. Das lineare Peptid kann zum Beispiel 1 oder 2 oder 3 oder 4 oder 5 oder 6 Lysine umfassen.

Beispiele für lineare Peptide sind EKE, EKEKE, EKEKEKE, EKEKEKEKE, EEE, EEEEE, EEEEEEE, EKEKEKEKEKE, EKEKEEKEKEK, EEEEEEEEK, eee, eeeee und eeeeeee, wobei E für L-Glutaminsäure, e für D-Glutaminsäure und K für L-Lysin steht.

In Formel (II) steht TX für eine Gruppe der Formel (III) oder (IV)

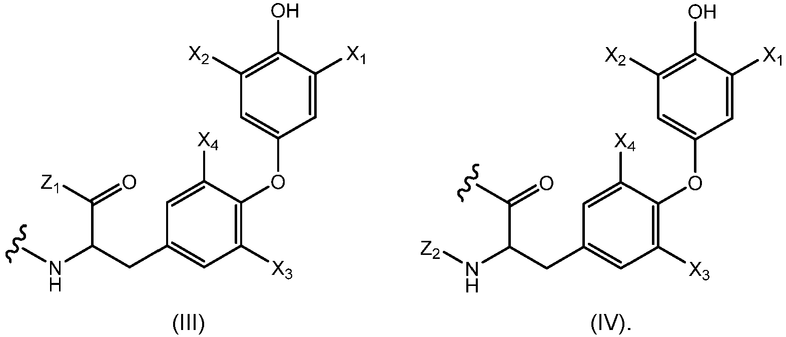

Darin stehen X₁, X₂, X₃ und X₄ unabhängig voneinander für I, H, Br, CI oder CN.

Zum Beispiel steht eines von X₁, X₂, X₃ und X₄ für H. Bevorzugt steht X₂ für H. Zum Beispiel stehen zwei von X₁, X₂, X₃ und X₄ für H, z.B. steht X₂ für H und X₄ für H.

Bevorzugt steht mindestens eines von X₁, X₂, X₃ und X₄ für I, weiter bevorzugt stehen mindestens zwei von X₁, X₂, X₃ und X₄ für I, noch weiter bevorzugt stehen drei von X₁, X₂, X₃ und X₄ für I.

Es ist im Rahmen der vorliegenden Erfindung bevorzugt, dass X₁, X₃ und X₄ für I stehen und X₂ für H steht.

Eines oder mehr als eines von X₁, X₂, X₃ und X₄ kann für Br, CI oder CN stehen. Bevorzugt steht nicht mehr als eines von X₁, X₂, X₃ und X₄ für Br, CI oder CN.

Z₁ steht für OH oder NH₂, und Z₂ steht für H oder C(O)CH₃. Bevorzugt steht Z₁ für NH₂.

Geeignete TX-Gruppen sind beispielsweise Gruppen der Formel (IIIa)-(IIIe) und (IVa)-(IVe):

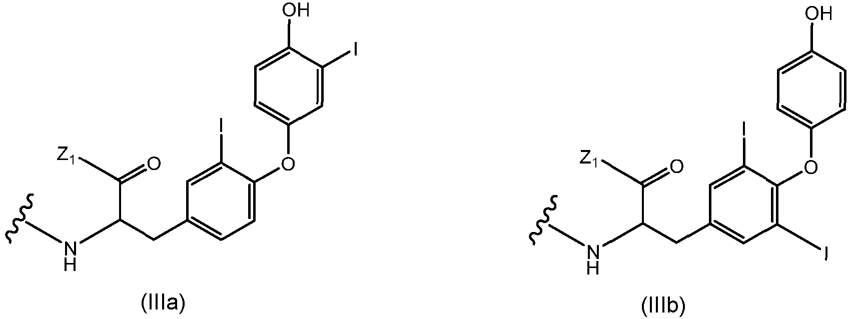

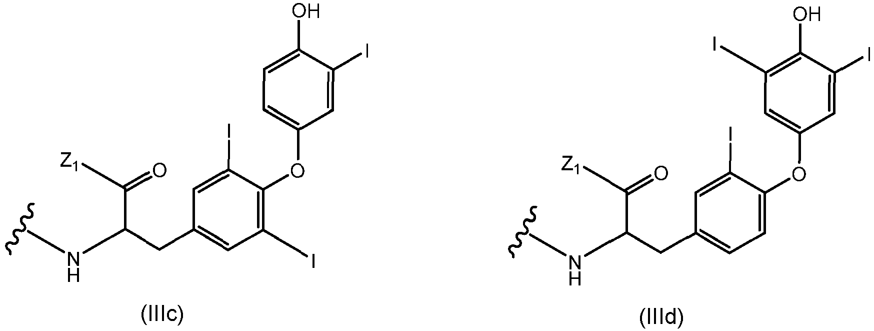

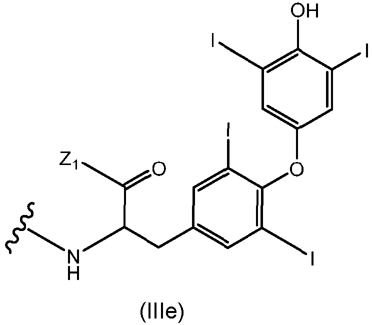

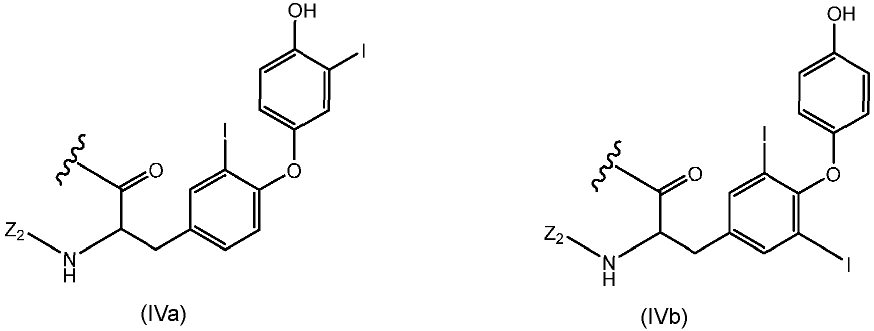

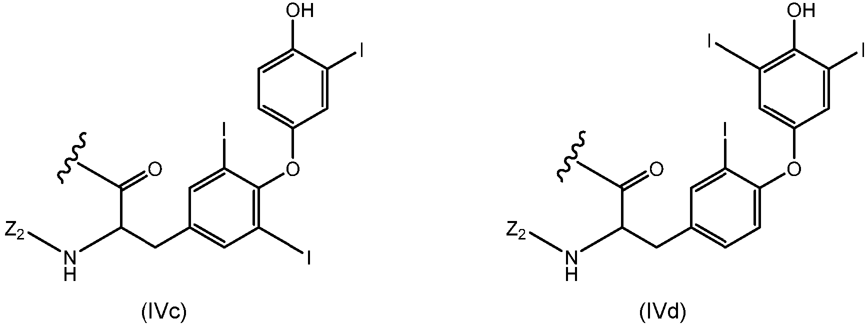

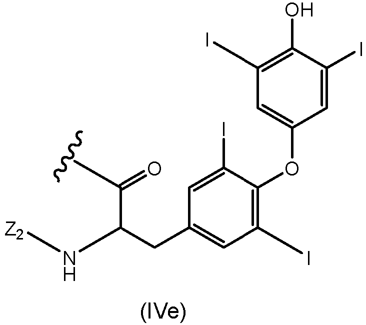

In Formel (II) steht CL für eine Chemilumineszenzgruppe. Die Chemilumineszenzgruppe basiert auf einem Chemilumineszenzfarbstoff, der über eine geeignete chemische Bindung mit dem linearen Peptid verknüpft werden kann. Bevorzugt ist die Chemilumineszenzgruppe am N-terminalen Ende des linearen Peptids gebunden, bevorzugt über eine Amidbindung.

Geeignete Chemilumineszenzfarbstoffe sind bevorzugt ausgewählt aus der Gruppe bestehend aus Acridiniumester-Farbstoffen und Acridiniumsulfonamid-Farbstoffen.

Als Beispiele für geeignete Chemilumineszenzfarbstoffe sind im Folgenden entweder die Chemilumineszenzfarbstoffe direkt oder ihre für die Kopplung geeigneten N-Hydroxysuccinimid-(NHS-)Aktivester angegeben:
3-(9-((3-Carboxypropyl)(tosyl)carbamoyl)acridinium-10-yl)propan-1-sulfonat (NSPSA, CAS-Nr. 211106-69-3, veröffentlicht in US5468646),
9-((4-(((2,5-Dioxopyrrolidin-1-yl)oxy)carbonyl)-2,6-dimethylphenoxy)carbonyl)-10-methylacridin-10-ium-methylsulfat (DMAE-NHS, CAS-Nr. 115853-74-2),
2',6'-Dimethylcarbonylphenyl-10-sulfopropylacridinium-9-carboxylat-4'-NHS-ester (NSP-DMAE-NHS, veröffentlicht in US5656426),
3-(9-((4-((23-((2,5-dioxopyrrolidin-1-yl)oxy)-19,23-dioxo-3,6,9,12, 15-pentaoxa-18-azatricosyl)carbamoyl)-2,6-dimethylphenoxy)carbonyl)acridin-10-ium-10-yl)propan-1-sulfonat (NSP-DMAE-HEG-Glu-NHS, veröffentlicht in US6664043B2),
2',6'-Dimethyl-4'-carboxyphenyl-2,7-bis[O-methoxyhexa(ethylen)glycol]-10-N-sulfopropyl-acridinium-9-carboxylat (NSP-2,7-(OMHEG)2-DMAE, veröffentlicht in US2005221390A1 / US7309615B2),
2',6'-Dimethyl-4'-carboxyphenyl-2,7-bis[O-methoxyhexa(ethylen)glycol]-10-N-methyl-acridinium-9-carboxylat (2,7-(OMHEG)2-DMAE, veröffentlicht in US2005221390A1 / US7309615B2),
2',6'-Dimethyl-4'-carboxyphenyl-2,7-bis[O-methoxytri(ethylen)glycol]-10-N-sulfopropyl-acridinium-9-carboxylat (NSP-2,7-(OMTEG)2-DMAE, veröffentlicht in US2005221390A1 / US7309615B2),
2'6'-Dimethyl-4'-N-succinimidyl-oxyglutarylamidohexa(ethylen)-glycolamido-carbonylphenyl-2,7-bis[O-methoxytri(ethylen)glycol]-10-sulfopropyl-acridinium-9-carboxylat (NSP-2,7-(OMTEG)2-DMAE-HEG-glutarate-NHS, veröffentlicht in US2005221390A1 / US7309615B2),
2',6'-Dimethyl-4'-N-succinimidyl-oxycaproylamidocarbonylphenyl-2,7-bis[O-methoxy(hexa)ethylenglycol]-10-N-sulfopropyl-acridinium-9-carboxylat (NSP-2,7-(OMHEG)2-DMAE-AC-NHS, veröffentlicht in US2005221390A1 / US7309615B2),
2',6'-Dimethyl-4'-N-succinimidyloxy-glutarylamidohexa(ethylen)glycolamidocarbonylphenyl-2,7-bis[O-methoxy-hexa(ethylen)glycol]-10-sulfopropyl-acridinium-9-carboxylat (NSP-2,7-(OMHEG)2-DMAE-HEG-glutarate-NHS, veröffentlicht in US2005221390A1 / US7309615B2),
4-(2-Succinimidyl-oxycarbonylethyl)-phenyl-10-acridinium-9-carboxylat-trifluormethylsulfonat (Acridinium C2-NHS, CAS-Nr. 177332-37-5),
9-Acridincarbonsäure-4-[3-[(2,5-dioxo-1-pyrrolidinyl)oxy]-3-oxopropyl]phenylester (Desmethyl Acridinium-NHS, CAS-Nr. 87198-87-6),
3-(9-((6-(2,5-Dioxopyrrolidin-1-yloxy)-6-oxohexyl)(tosyl)carbamoyl)acridinium-10-yl)propan-1-sulfonat (FD106521-NHS, CAS-Nr. 866366-12-3),
10-Methyl-N-butyl-N-p-toluolsulfonyl-9-acridiniumcarboxamid (veröffentlicht in US5468646),
10-Methyl-N-(5-carboxypentyl)-N-p-nitrobenzolsulfonyl-9-acridiniumcarboxamid (veröffentlicht in US5468646),
10-(3-Sulfopropyl)-N-tosyl-N-(3-sulfopropyl)-9-acridiniumcarboxamid (veröffentlicht in US5468646), 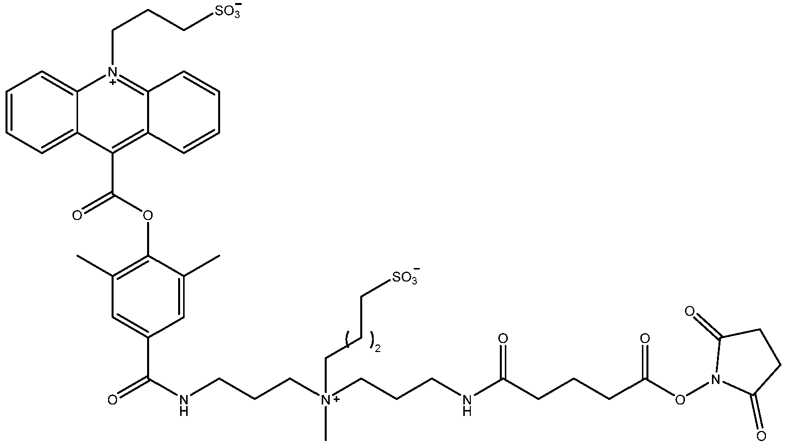 (NSP-DMAE-Z-NHS, veröffentlicht in US8778624B2).

Besonders bevorzugt basiert die Chemilumineszenzgruppe auf 3-(9-((3-Carboxypropyl)(tosyl)carbamoyl)acridinium-10-yl)propan-1-sulfonat (NSPSA).

Beispiele für T3-basierte Thyronin-Derivat-Tracer der Formel (II) sind im Folgenden in Form der Tracer-Kurzformel gegeben:
NSPSA-EEEE-(lllc)OH, NSPSA-EEEEE-(lllc)OH, NSPSA-EEEEEE-(lllc)OH, NSPSA-EEEEEEE-(lllc)OH, NSPSA-EEEEEEEE-(lllc)OH, NSPSA-EEEE-(IIIc)NH₂, NSPSA-EEEEE-(IIIc)NH₂, NSPSA-EEEEEE-(IIIc)NH₂, NSPSA-EEEEEEE-(IIIc)NH₂, NSPSA-EEEEEEEE-(IIIc)NH₂, NSPSA-eeee-(IIIc)NH₂, NSPSA-eeeee-(IIIc)NH₂, NSPSA-eeeeee-(IIIc)NH₂, NSPSA-eeeeeee-(IIIc)NH₂, NSPSA-eeeeeeee-(IIIc)NH₂, NSPSA-eeeeeeeee-(IIIc)NH₂,
NSPSA-eeee-(lllc)OH, NSPSA-eeeee-(lllc)OH, NSPSA-eeeeee-(lllc)OH, NSPSA-eeeeeee-(lllc)OH, NSPSA-eeeeeeee-(lllc)OH, NSPSA-eeeeeeeee-(lllc)OH, NSP-DMAE-HEG-Glu-EEEE-(lllc)OH, NSP-DMAE-HEG-Glu-EEEEE-(lllc)OH, NSP-DMAE-HEG-Glu-EEEEEE-(lllc)OH, NSP-DMAE-HEG-Glu-EEEEEEE-(lllc)OH, NSP-DMAE-HEG-Glu-EEEEEEEE-(lllc)OH, NSP-DMAE-HEG-Glu-EEEE-(IIIc)NH₂, NSP-DMAE-HEG-Glu-EEEEE-(IIIc)NH₂, NSP-DMAE-HEG-Glu-EEEEEE-(IIIc)NH₂, NSP-DMAE-HEG-Glu-EEEEEEE-(IIIc)NH₂, NSP-DMAE-HEG-Glu-EEEEEEEE-(IIIc)NH₂, NSP-DMAE-H EG-Glu-eeee-(IIIc)NH₂, NSP-DMAE-HEG-Glu-eeeee-(IIIc)NH₂, NSP-DMAE-HEG-Glu-eeeeee-(IIIc)NH₂, NSP-DMAE-HEG-Glu-eeeeeee-(IIIc)NH₂, NSP-DMAE-HEG-Glu-eeeeeeee-(IIIc)NH₂, NSP-DMAE-HEG-Glu-eeeeeeeee-(IIIc)NH₂,
NSP-DMAE-HEG-Glu-eeee-(lllc)OH, NSP-DMAE-HEG-Glu-eeeee-(lllc)OH, NSP-DMAE-HEG-Glu-eeeeee-(lllc)OH, NSP-DMAE-HEG-Glu-eeeeeee-(lllc)OH, NSP-DMAE-HEG-Glu-eeeeeeee-(lllc)OH, NSP-DMAE-HEG-Glu-eeeeeeeee-(lllc)OH,
NSP-DMAE-EEEE-(lllc)OH, NSP-DMAE-EEEEE-(lllc)OH, NSP-DMAE-EEEEEE-(IIIc)OH, NSP-DMAE-EEEEEEE-(lllc)OH, NSP-DMAE-EEEEEEEE-(lllc)OH, NSP-DMAE-EEEE-(IIIc)NH₂, NSP-DMAE-EEEEE-(IIIc)NH₂, NSP-DMAE-EEEEEE-(IIIc)NH₂, NSP-DMAE-EEEEEEE-(IIIc)NH₂, NSP-DMAE-EEEEEEEE-(IIIc)NH₂,
NSP-DMAE-eeee-(IIIc)NH₂, NSP-DMAE-eeeee-(IIIc)NH₂, NSP-DMAE-eeeeee-(IIIc)NH₂, NSP-DMAE-eeeeeee-(IIIc)NH₂, NSP-DMAE-eeeeeeee-(IIIc)NH₂, NSP-DMAE-eeeeeeeee-(IIIc)NH₂,
NSP-DMAE-eeee-(lllc)OH, NSP-DMAE-eeeee-(lllc)OH, NSP-DMAE-eeeeee-(lllc)OH, NSP-DMAE-eeeeeee-(lllc)OH, NSP-DMAE-eeeeeeee-(lllc)OH, NSP-DMAE-eeeeeeeee-(IIIc)OH,
NSP-2,7-(OMHEG)2-DMAE-EEEE-(IIIc)OH, NSP-2,7-(OMHEG)2-DMAE-EEEEE-(IIIc)OH, NSP-2,7-(OMHEG)2-DMAE-EEEEEE-(lllc)OH, NSP-2,7-(OMHEG)2-DMAE-EEEEEEE-(lllc)OH, NSP-2,7-(OMHEG)2-DMAE-EEEEEEEE-(lllc)OH, NSP-2,7-(OMHEG)2-DMAE-EEEE-(IIIc)NH₂, NSP-2,7-(OMHEG)2-DMAE-EEEEE-(IIIc)NH₂, NSP-2,7-(OMHEG)2-DMAE-EEEEEE-(IIIc)NH₂, NSP-2,7-(OMHEG)2-DMAE-EEEEEEE-(IIIc)NH₂, NSP-2,7-(OMHEG)2-DMAE-EEEEEEEE-(IIIc)NH₂, NSP-2,7-(OMHEG)2-DMAE-eeee-(IIIc)NH₂, NSP-2,7-(OMHEG)2-DMAE-eeeee-(IIIc)NH₂, NSP-2,7-(OMHEG)2-DMAE-eeeeee-(IIIc)NH₂, NSP-2,7-(OMHEG)2-DMAE-eeeeeee-(IIIc)NH₂, NSP-2,7-(OMHEG)2-DMAE-eeeeeeee-(IIIc)NH₂, NSP-2,7-(OMHEG)2-DMAE-eeeeeeeee-(IIIc)NH₂,
NSP-2,7-(OMHEG)2-DMAE-eeee-(lllc)OH, NSP-2,7-(OMHEG)2-DMAE-eeeee-(lllc)OH, NSP-2,7-(OMHEG)2-DMAE-eeeeee-(lllc)OH, NSP-2,7-(OMHEG)2-DMAE-eeeeeee-(IIIc)OH, NSP-2,7-(OMHEG)2-DMAE-eeeeeeee-(lllc)OH, NSP-2,7-(OMHEG)2-DMAE-eeeeeeeee-(lllc)OH,
NSPSA-EEEEK-(lVc)H, NSPSA-EEEEEK-(lVc)H, NSPSA-EEEEEEK-(lVc)H, NSPSA-EEEEEEEK-(lVc)H, NSPSA-EEEEEEEEK-(lVc)H, NSPSA-eeeeK-(IVc)C(O)CH3, NSPSA-eeeeeK-(IVc)C(O)CH3, NSPSA-eeeeeeK-(IVc)C(O)CH3, NSPSA-eeeeeeeK-(IVc)C(O)CH3, NSPSA-eeeeeeeeK-(IVc)C(O)CH3, NSPSA-eeeeeeeeeK-(IVc)C(O)CH3, NSPSA-eeeeK-(lVc)H, NSPSA-eeeeeK-(IVc)H, NSPSA-eeeeeeK-(lVc)H, NSPSA-eeeeeeeK-(IVc)H, NSPSA-eeeeeeeeK-(IVc)H, NSPSA-eeeeeeeeeK-(IVc)H,
NSP-DMAE-HEG-Glu-EEEEK-(lVc)H, NSP-DMAE-HEG-Glu-EEEEEK-(lVc)H, NSP-DMAE-HEG-Glu-EEEEEEK-(IVc)H, NSP-DMAE-HEG-Glu-EEEEEEEK-(lVc)H, NSP-DMAE-HEG-Glu-EEEEEEEEK-(IVc)H, NSP-DMAE-HEG-Glu-eeeeK-(IVc)C(O)CH3, NSP-DMAE-HEG-Glu-eeeeeK-(IVc)C(O)CH3, NSP-DMAE-HEG-Glu-eeeeeeK-(IVc)C(O)CH3, NSP-DMAE-HEG-Glu-eeeeeeeK-(IVc)C(O)CH3, NSP-DMAE-HEG-GlueeeeeeeeK-(IVc)C(O)CH3, NSP-DMAE-HEG-Glu-eeeeeeeeeK-(IVc)C(O)CH3,
NSP-DMAE-HEG-Glu-eeeeK-(IVc)H, NSP-DMAE-HEG-Glu-eeeeeK-(IVc)H, NSP-DMAE-HEG-Glu-eeeeeeK-(lVc)H, NSP-DMAE-HEG-Glu-eeeeeeeK-(IVc)H, NSP-DMAE-HEG-Glu-eeeeeeeeK-(IVc)H, NSP-DMAE-HEG-Glu-eeeeeeeeeK-(IVc)H,
NSP-DMAE-EEEEK-(lVc)H, NSP-DMAE-EEEEEK-(lVc)H, NSP-DMAE-EEEEEEK-(IVc)H, NSP-DMAE-EEEEEEEK-(IVc)H, NSP-DMAE-EEEEEEEEK-(IVc)H, NSP-DMAE-eeeeK-(IVc)C(O)CH3, NSP-DMAE-eeeeeK-(IVc)C(O)CH3, NSP-DMAE-eeeeeeK-(IVc)C(O)CH3, NSP-DMAE-eeeeeeeK-(IVc)C(O)CH3, NSP-DMAE-eeeeeeeeK-(IVc)C(O)CH3, NSP-DMAE-eeeeeeeeeK-(IVc)C(O)CH3,
NSP-DMAE-eeeeK-(IVc)H, NSP-DMAE-eeeeeK-(IVc)H, NSP-DMAE-eeeeeeK-(IVc)H, NSP-DMAE-eeeeeeeK-(IVc)H, NSP-DMAE-eeeeeeeeK-(IVc)H, NSP-DMAE-eeeeeeeeeK-(lVc)H,
NSP-2,7-(OMHEG)2-DMAE-EEEEK-(IVc)H, NSP-2,7-(OMHEG)2-DMAE-EEEEEK-(IVc)H, NSP-2,7-(OMHEG)2-DMAE-EEEEEEK-(IVc)H, NSP-2,7-(OMHEG)2-DMAE-EEEEEEEK-(lVc)H, NSP-2,7-(OMHEG)2-DMAE-EEEEEEEEK-(IVc)H, NSP-2,7-(OMHEG)2-DMAE-eeeeK-(IVc)C(O)CH3, NSP-2,7-(OMHEG)2-DMAE-eeeeeK-(IVc)C(O)CH3, NSP-2,7-(OMHEG)2-DMAE-eeeeeeK-(IVc)C(O)CH3, NSP-2,7-(OMHEG)2-DMAE-eeeeeeeK-(IVc)C(O)CH3, NSP-2,7-(OMHEG)2-DMAE-eeeeeeeeK-(IVc)C(O)CH3, NSP-2,7-(OMHEG)2-DMAE-eeeeeeeeeK-(IVc)C(O)CH3,
NSP-2,7-(OMHEG)2-DMAE-eeeeK-(IVc)H, NSP-2,7-(OMHEG)2-DMAE-eeeeeK-(IVc)H, NSP-2,7-(OMHEG)2-DMAE-eeeeeeK-(IVc)H, NSP-2,7-(OMHEG)2-DMAE-eeeeeeeK-(IVc)H, NSP-2,7-(OMHEG)2-DMAE-eeeeeeeeK-(IVc)H, NSP-2,7-(OMHEG)2-DMAE-eeeeeeeeeK-(lVc)H.

Beispiele für rT3-basierte Thyronin-Derivat-Tracer der Formel (II) sind im Folgenden in Form der Tracer-Kurzformel gegeben:
NSPSA-EEEE-(Illd)OH, NSPSA-EEEEE-(Illd)OH, NSPSA-EEEEEE-(Illd)OH, NSPSA-EEEEEEE-(Illd)OH, NSPSA-EEEEEEEE-(llld)OH, NSPSA-EEEE-(IIId)NH₂, NSPSA-EEEEE-(IIId)NH₂, NSPSA-EEEEEE-(IIId)NH₂, NSPSA-EEEEEEE-(IIId)NH₂, NSPSA-EEEEEEEE-(IIId)NH₂, NSPSA-eeee-(IIId)NH₂, NSPSA-eeeee-(IIId)NH₂, NSPSA-eeeeee-(IIId)NH₂, NSPSA-eeeeeee-(IIId)NH₂, NSPSA-eeeeeeee-(IIId)NH₂, NSPSA-eeeeeeeee-(IIId)NH₂,
NSPSA-eeee-(Illd)OH, NSPSA-eeeee-(Illd)OH, NSPSA-eeeeee-(Illd)OH, NSPSA-eeeeeee-(Illd)OH, NSPSA-eeeeeeee-(Illd)OH, NSPSA-eeeeeeeee-(Illd)OH,
NSP-DMAE-HEG-Glu-EEEE-(Illd)OH, NSP-DMAE-HEG-Glu-EEEEE-(Illd)OH, NSP-DMAE-HEG-Glu-EEEEEE-(Illd)OH, NSP-DMAE-HEG-Glu-EEEEEEE-(Illd)OH, NSP-DMAE-HEG-Glu-EEEEEEEE-(Illd)OH, NSP-DMAE-HEG-Glu-EEEE-(IIId)NH₂, NSP-DMAE-HEG-Glu-EEEEE-(IIId)NH₂, NSP-DMAE-HEG-Glu-EEEEEE-(IIId)NH₂, NSP-DMAE-HEG-Glu-EEEEEEE-(Illd)NH2, NSP-DMAE-HEG-Glu-EEEEEEEE-(Illd)NH2,
NSP-DMAE-HEG-Glu-eeee-(Illd)NH2, NSP-DMAE-HEG-Glu-eeeee-(Illd)NH2, NSP-DMAE-HEG-Glu-eeeeee-(Illd)NH2, NSP-DMAE-HEG-Glu-eeeeeee-(Illd)NH2, NSP-DMAE-HEG-Glu-eeeeeeee-(IIId)NH₂, NSP-DMAE-HEG-Glu-eeeeeeeee-(IIId)NH₂,
NSP-DMAE-HEG-Glu-eeee-(Illd)OH, NSP-DMAE-HEG-Glu-eeeee-(Illd)OH, NSP-DMAE-HEG-Glu-eeeeee-(Illd)OH, NSP-DMAE-HEG-Glu-eeeeeee-(Illd)OH, NSP-DMAE-HEG-Glu-eeeeeeee-(Illd)OH, NSP-DMAE-HEG-Glu-eeeeeeeee-(Illd)OH,
NSP-DMAE-EEEE-(Illd)OH, NSP-DMAE-EEEEE-(Illd)OH, NSP-DMAE-EEEEEE-(IIId)OH, NSP-DMAE-EEEEEEE-(Illd)OH, NSP-DMAE-EEEEEEEE-(llld)OH, NSP-DMAE-EEEE-(Illd)NH2, NSP-DMAE-EEEEE-(Illd)NH2, NSP-DMAE-EEEEEE-(Illd)NH2, NSP-DMAE-EEEEEEE-(Illd)NH2, NSP-DMAE-EEEEEEEE-(llld)NH2,
NSP-DMAE-eeee-(Illd)NH2, NSP-DMAE-eeeee-(Illd)NH2, NSP-DMAE-eeeeee-(IIId)NH2, NSP-DMAE-eeeeeee-(Illd)NH2, NSP-DMAE-eeeeeeee-(Illd)NH2, NSP-DMAE-eeeeeeeee-(Illd)NH2,
NSP-DMAE-eeee-(Illd)OH, NSP-DMAE-eeeee-(Illd)OH, NSP-DMAE-eeeeee-(Illd)OH, NSP-DMAE-eeeeeee-(Illd)OH, NSP-DMAE-eeeeeeee-(Illd)OH, NSP-DMAE-eeeeeeeee-(IIId)OH,
NSP-2,7-(OMHEG)2-DMAE-EEEE-(IIId)OH, NSP-2,7-(OMHEG)2-DMAE-EEEEE-(IIId)OH, NSP-2,7-(OMHEG)2-DMAE-EEEEEE-(Illd)OH, NSP-2,7-(OMHEG)2-DMAE-EEEEEEE-(Illd)OH, NSP-2,7-(OMHEG)2-DMAE-EEEEEEEE-(Illd)OH, NSP-2,7-(OMHEG)2-DMAE-EEEE-(IIId)NH₂, NSP-2,7-(OMHEG)2-DMAE-EEEEE-(IIId)NH₂, NSP-2,7-(OMHEG)2-DMAE-EEEEEE-(IIId)NH₂, NSP-2,7-(OMHEG)2-DMAE-EEEEEEE-(IIId)NH₂, NSP-2,7-(OMHEG)2-DMAE-EEEEEEEE-(IIId)NH₂, NSP-2,7-(OMHEG)2-DMAE-eeee-(IIId)NH₂, NSP-2,7-(OMHEG)2-DMAE-eeeee-(IIId)NH₂, NSP-2,7-(OMHEG)2-DMAE-eeeeee-(IIId)NH₂, NSP-2,7-(OMHEG)2-DMAE-eeeeeee-(IIId)NH₂, NSP-2,7-(OMHEG)2-DMAE-eeeeeeee-(IIId)NH₂, NSP-2,7-(OMHEG)2-DMAE-eeeeeeeee-(IIId)NH₂,
NSP-2,7-(OMHEG)2-DMAE-eeee-(IIId)OH, NSP-2,7-(OMHEG)2-DMAE-eeeee-(IIId)OH, NSP-2,7-(OMHEG)2-DMAE-eeeeee-(IIId)OH, NSP-2,7-(OMHEG)2-DMAE-eeeeeee-(IIId)OH, NSP-2,7-(OMHEG)2-DMAE-eeeeeeee-(Illd)OH, NSP-2,7-(OMHEG)2-DMAE-eeeeeeeee-(Illd)OH,
NSPSA-EEEEK-(IVd)H, NSPSA-EEEEEK-(IVd)H, NSPSA-EEEEEEK-(IVd)H, NSPSA-EEEEEEEK-(IVd)H, NSPSA-EEEEEEEEK-(IVd)H, NSPSA-eeeeK-(IVd)C(O)CH3, NSPSA-eeeeeK-(IVd)C(O)CH3, NSPSA-eeeeeeK-(IVd)C(O)CH3, NSPSA-eeeeeeeK-(IVd)C(O)CH3, NSPSA-eeeeeeeeK-(IVd)C(O)CH3, NSPSA-eeeeeeeeeK-(IVd)C(O)CH3, NSPSA-eeeeK-(IVd)H, NSPSA-eeeeeK-(IVd)H, NSPSA-eeeeeeK-(IVd)H, NSPSA-eeeeeeeK-(IVd)H, NSPSA-eeeeeeeeK-(IVd)H, NSPSA-eeeeeeeeeK-(IVd)H,
NSP-DMAE-HEG-Glu-EEEEK-(IVd)H, NSP-DMAE-HEG-Glu-EEEEEK-(IVd)H, NSP-DMAE-HEG-Glu-EEEEEEK-(IVd)H, NSP-DMAE-HEG-Glu-EEEEEEEK-(IVd)H, NSP-DMAE-HEG-Glu-EEEEEEEEK-(IVd)H, NSP-DMAE-HEG-Glu-eeeeK-(IVd)C(O)CH3, NSP-DMAE-HEG-Glu-eeeeeK-(IVd)C(O)CH3, NSP-DMAE-HEG-Glu-eeeeeeK-(IVd)C(O)CH3, NSP-DMAE-HEG-Glu-eeeeeeeK-(IVd)C(O)CH3, NSP-DMAE-HEG-Glu-eeeeeeeeK-(IVd)C(O)CH3, NSP-DMAE-HEG-Glu-eeeeeeeeeK-(IVd)C(O)CH3,
NSP-DMAE-HEG-Glu-eeeeK-(IVd)H, NSP-DMAE-HEG-Glu-eeeeeK-(IVd)H, NSP-DMAE-HEG-Glu-eeeeeeK-(IVd)H, NSP-DMAE-HEG-Glu-eeeeeeeK-(IVd)H, NSP-DMAE-HEG-Glu-eeeeeeeeK-(IVd)H, NSP-DMAE-HEG-Glu-eeeeeeeeeK-(IVd)H,
NSP-DMAE-EEEEK-(IVd)H, NSP-DMAE-EEEEEK-(IVd)H, NSP-DMAE-EEEEEEK-(IVd)H, NSP-DMAE-EEEEEEEK-(IVd)H, NSP-DMAE-EEEEEEEEK-(IVd)H, NSP-DMAE-eeeeK-(IVd)C(O)CH3, NSP-DMAE-eeeeeK-(IVd)C(O)CH3, NSP-DMAE-eeeeeeK-(IVd)C(O)CH3, NSP-DMAE-eeeeeeeK-(IVd)C(O)CH3, NSP-DMAE-eeeeeeeeK-(IVd)C(O)CH3, NSP-DMAE-eeeeeeeeeK-(IVd)C(O)CH3,
NSP-DMAE-eeeeK-(IVd)H, NSP-DMAE-eeeeeK-(IVd)H, NSP-DMAE-eeeeeeK-(IVd)H, NSP-DMAE-eeeeeeeK-(IVd)H, NSP-DMAE-eeeeeeeeK-(IVd)H, NSP-DMAE-eeeeeeeeeK-(IVd)H,
NSP-2,7-(OMHEG)2-DMAE-EEEEK-(IVd)H, NSP-2,7-(OMHEG)2-DMAE-EEEEEK-(IVd)H, NSP-2,7-(OMHEG)2-DMAE-EEEEEEK-(IVd)H, NSP-2,7-(OMHEG)2-DMAE-EEEEEEEK-(IVd)H, NSP-2,7-(OMHEG)2-DMAE-EEEEEEEEK-(IVd)H, NSP-2,7-(OMHEG)2-DMAE-eeeeK-(IVd)C(O)CH3, NSP-2,7-(OMHEG)2-DMAE-eeeeeK-(IVd)C(O)CH3, NSP-2,7-(OMHEG)2-DMAE-eeeeeeK-(IVd)C(O)CH3, NSP-2,7-(OMHEG)2-DMAE-eeeeeeeK-(IVd)C(O)CH3, NSP-2,7-(OMHEG)2-DMAE-eeeeeeeeK-(IVd)C(O)CH3, NSP-2,7-(OMHEG)2-DMAE-eeeeeeeeeK-(IVd)C(O)CH3,
NSP-2,7-(OMHEG)2-DMAE-eeeeK-(IVd)H, NSP-2,7-(OMHEG)2-DMAE-eeeeeK-(IVd)H, NSP-2,7-(OMHEG)2-DMAE-eeeeeeK-(IVd)H, NSP-2,7-(OMHEG)2-DMAE-eeeeeeeK-(IVd)H, NSP-2,7-(OMHEG)2-DMAE-eeeeeeeeK-(IVd)H, NSP-2,7-(OMHEG)2-DMAE-eeeeeeeeeK-(IVd)H.

Beispiele für T2-basierte Thyronin-Derivat-Tracer der Formel (II) sind im Folgenden in Form der Tracer-Kurzformel gegeben:
NSPSA-EEEE-(Illa)OH, NSPSA-EEEEE-(Illa)OH, NSPSA-EEEEEE-(Illa)OH, NSPSA-EEEEEEE-(Illa)OH, NSPSA-EEEEEEEE-(Illa)OH, NSPSA-EEEE-(IIIa)NH₂, NSPSA-EEEEE-(IIIa)NH₂, NSPSA-EEEEEE-(IIIa)NH₂, NSPSA-EEEEEEE-(IIIa)NH₂, NSPSA-EEEEEEEE-(IIIa)NH₂, NSPSA-eeee-(IIIa)NH₂, NSPSA-eeeee-(IIIa)NH₂, NSPSA-eeeeee-(IIIa)NH₂, NSPSA-eeeeeee-(IIIa)NH₂, NSPSA-eeeeeeee-(IIIa)NH₂, NSPSA-eeeeeeeee-(IIIa)NH₂,
NSPSA-eeee-(Illa)OH, NSPSA-eeeee-(Illa)OH, NSPSA-eeeeee-(Illa)OH, NSPSA-eeeeeee-(Illa)OH, NSPSA-eeeeeeee-(Illa)OH, NSPSA-eeeeeeeee-(Illa)OH,
NSP-DMAE-HEG-Glu-EEEE-(Illa)OH, NSP-DMAE-HEG-Glu-EEEEE-(Illa)OH, NSP-DMAE-HEG-Glu-EEEEEE-(Illa)OH, NSP-DMAE-HEG-Glu-EEEEEEE-(Illa)OH, NSP-DMAE-HEG-Glu-EEEEEEEE-(Illa)OH, NSP-DMAE-HEG-Glu-EEEE-(IIIa)NH₂, NSP-DMAE-HEG-Glu-EEEEE-(Illa)NH2, NSP-DMAE-HEG-Glu-EEEEEE-(Illa)NH2, NSP-DMAE-HEG-Glu-EEEEEEE-(Illa)NH2, NSP-DMAE-HEG-Glu-EEEEEEEE-(Illa)NH2,
NSP-DMAE-HEG-Glu-eeee-(Illa)NH2, NSP-DMAE-HEG-Glu-eeeee-(Illa)NH2, NSP-DMAE-HEG-Glu-eeeeee-(Illa)NH2, NSP-DMAE-HEG-Glu-eeeeeee-(Illa)NH2, NSP-DMAE-HEG-Glu-eeeeeeee-(Illa)NH2, NSP-DMAE-HEG-Glu-eeeeeeeee-(Illa)NH2,
NSP-DMAE-HEG-Glu-eeee-(Illa)OH, NSP-DMAE-HEG-Glu-eeeee-(Illa)OH, NSP-DMAE-HEG-Glu-eeeeee-(Illa)OH, NSP-DMAE-HEG-Glu-eeeeeee-(Illa)OH, NSP-DMAE-HEG-Glu-eeeeeeee-(Illa)OH, NSP-DMAE-HEG-Glu-eeeeeeeee-(Illa)OH,
NSP-DMAE-EEEE-(Illa)OH, NSP-DMAE-EEEEE-(Illa)OH, NSP-DMAE-EEEEEE-(IIIa)OH, NSP-DMAE-EEEEEEE-(Illa)OH, NSP-DMAE-EEEEEEEE-(Illa)OH, NSP-DMAE-EEEE-(IIIa)NH₂, NSP-DMAE-EEEEE-(IIIa)NH₂, NSP-DMAE-EEEEEE-(IIIa)NH₂, NSP-DMAE-EEEEEEE-(Illa)NH2, NSP-DMAE-EEEEEEEE-(Illa)NH2,
NSP-DMAE-eeee-(Illa)NH2, NSP-DMAE-eeeee-(Illa)NH2, NSP-DMAE-eeeeee-(IIIa)NH2, NSP-DMAE-eeeeeee-(Illa)NH2, NSP-DMAE-eeeeeeee-(Illa)NH2, NSP-DMAE-eeeeeeeee-(IIIa)NH₂,
NSP-DMAE-eeee-(Illa)OH, NSP-DMAE-eeeee-(Illa)OH, NSP-DMAE-eeeeee-(Illa)OH, NSP-DMAE-eeeeeee-(Illa)OH, NSP-DMAE-eeeeeeee-(Illa)OH, NSP-DMAE-eeeeeeeee-(IIIa)OH,
NSP-2,7-(OMHEG)2-DMAE-EEEE-(Illa)OH, NSP-2,7-(OMHEG)2-DMAE-EEEEE-(IIIa)OH, NSP-2,7-(OMHEG)2-DMAE-EEEEEE-(Illa)OH, NSP-2,7-(OMHEG)2-DMAE-EEEEEEE-(Illa)OH, NSP-2,7-(OMHEG)2-DMAE-EEEEEEEE-(Illa)OH, NSP-2,7-(OMHEG)2-DMAE-EEEE-(IIIa)NH₂, NSP-2,7-(OMHEG)2-DMAE-EEEEE-(IIIa)NH₂, NSP-2,7-(OMHEG)2-DMAE-EEEEEE-(IIIa)NH₂, NSP-2,7-(OMHEG)2-DMAE-EEEEEEE-(IIIa)NH₂, NSP-2,7-(OMHEG)2-DMAE-EEEEEEEE-(IIIa)NH₂, NSP-2,7-(OMHEG)2-DMAE-eeee-(IIIa)NH₂, NSP-2,7-(OMHEG)2-DMAE-eeeee-(IIIa)NH₂, NSP-2,7-(OMHEG)2-DMAE-eeeeee-(IIIa)NH₂, NSP-2,7-(OMHEG)2-DMAE-eeeeeee-(IIIa)NH₂, NSP-2,7-(OMHEG)2-DMAE-eeeeeeee-(IIIa)NH₂, NSP-2,7-(OMHEG)2-DMAE-eeeeeeeee-(IIIa)NH₂,
NSP-2,7-(OMHEG)2-DMAE-eeee-(Illa)OH, NSP-2,7-(OMHEG)2-DMAE-eeeee-(Illa)OH, NSP-2,7-(OMHEG)2-DMAE-eeeeee-(Illa)OH, NSP-2,7-(OMHEG)2-DMAE-eeeeeee-(IIIa)OH, NSP-2,7-(OMHEG)2-DMAE-eeeeeeee-(Illa)OH, NSP-2,7-(OMHEG)2-DMAE-eeeeeeeee-(Illa)OH,
NSPSA-EEEEK-(IVa)H, NSPSA-EEEEEK-(IVa)H, NSPSA-EEEEEEK-(IVa)H, NSPSA-EEEEEEEK-(IVa)H, NSPSA-EEEEEEEEK-(IVa)H, NSPSA-eeeeK-(IVa)C(O)CH3, NSPSA-eeeeeK-(IVa)C(O)CH3, NSPSA-eeeeeeK-(IVa)C(O)CH3, NSPSA-eeeeeeeK-(IVa)C(O)CH3, NSPSA-eeeeeeeeK-(IVa)C(O)CH3, NSPSA-eeeeeeeeeK-(IVa)C(O)CH3, NSPSA-eeeeK-(IVa)H, NSPSA-eeeeeK-(IVa)H, NSPSA-eeeeeeK-(IVa)H, NSPSA-eeeeeeeK-(IVa)H, NSPSA-eeeeeeeeK-(IVa)H, NSPSA-eeeeeeeeeK-(IVa)H,
NSP-DMAE-HEG-Glu-EEEEK-(IVa)H, NSP-DMAE-HEG-Glu-EEEEEK-(IVa)H, NSP-DMAE-HEG-Glu-EEEEEEK-(IVa)H, NSP-DMAE-HEG-Glu-EEEEEEEK-(IVa)H, NSP-DMAE-HEG-Glu-EEEEEEEEK-(IVa)H, NSP-DMAE-HEG-Glu-eeeeK-(IVa)C(O)CH3, NSP-DMAE-HEG-Glu-eeeeeK-(IVa)C(O)CH3, NSP-DMAE-HEG-Glu-eeeeeeK-(IVa)C(O)CH3, NSP-DMAE-HEG-Glu-eeeeeeeK-(IVa)C(O)CH3, NSP-DMAE-HEG-Glu-eeeeeeeeK-(IVa)C(O)CH3, NSP-DMAE-HEG-Glu-eeeeeeeeeK-(IVa)C(O)CH3,
NSP-DMAE-HEG-Glu-eeeeK-(IVa)H, NSP-DMAE-HEG-Glu-eeeeeK-(IVa)H, NSP-DMAE-HEG-Glu-eeeeeeK-(IVa)H, NSP-DMAE-HEG-Glu-eeeeeeeK-(IVa)H, NSP-DMAE-HEG-Glu-eeeeeeeeK-(IVa)H, NSP-DMAE-HEG-Glu-eeeeeeeeeK-(IVa)H,
NSP-DMAE-EEEEK-(IVa)H, NSP-DMAE-EEEEEK-(IVa)H, NSP-DMAE-EEEEEEK-(IVa)H, NSP-DMAE-EEEEEEEK-(!Va)H, NSP-DMAE-EEEEEEEEK-(IVa)H, NSP-DMAE-eeeeK-(IVa)C(O)CH3, NSP-DMAE-eeeeeK-(IVa)C(O)CH3, NSP-DMAE-eeeeeeK-(IVa)C(O)CH3, NSP-DMAE-eeeeeeeK-(IVa)C(O)CH3, NSP-DMAE-eeeeeeeeK-(IVa)C(O)CH3, NSP-DMAE-eeeeeeeeeK-(IVa)C(O)CH3,
NSP-DMAE-eeeeK-(IVa)H, NSP-DMAE-eeeeeK-(IVa)H, NSP-DMAE-eeeeeeK-(IVa)H, NSP-DMAE-eeeeeeeK-(IVa)H, NSP-DMAE-eeeeeeeeK-(IVa)H, NSP-DMAE-eeeeeeeeeK-(IVa)H,
NSP-2,7-(OMHEG)2-DMAE-EEEEK-(IVa)H, NSP-2,7-(OMHEG)2-DMAE-EEEEEK-(IVa)H, NSP-2,7-(OMHEG)2-DMAE-EEEEEEK-(IVa)H, NSP-2,7-(OMHEG)2-DMAE-EEEEEEEK-(IVa)H, NSP-2,7-(OMHEG)2-DMAE-EEEEEEEEK-(IVa)H, NSP-2,7-(OMHEG)2-DMAE-eeeeK-(IVa)C(O)CH3, NSP-2,7-(OMHEG)2-DMAE-eeeeeK-(IVa)C(O)CH3, NSP-2,7-(OMHEG)2-DMAE-eeeeeeK-(IVa)C(O)CH3, NSP-2,7-(OMHEG)2-DMAE-eeeeeeeK-(IVa)C(O)CH3, NSP-2,7-(OMHEG)2-DMAE-eeeeeeeeK-(IVa)C(O)CH3, NSP-2,7-(OMHEG)2-DMAE-eeeeeeeeeK-(IVa)C(O)CH3,
NSP-2,7-(OMHEG)2-DMAE-eeeeK-(IVa)H, NSP-2,7-(OMHEG)2-DMAE-eeeeeK-(IVa)H, NSP-2,7-(OMHEG)2-DMAE-eeeeeeK-(IVa)H, NSP-2,7-(OMHEG)2-DMAE-eeeeeeeK-(IVa)H, NSP-2,7-(OMHEG)2-DMAE-eeeeeeeeK-(IVa)H, NSP-2,7-(OMHEG)2-DMAE-eeeeeeeeeK-(IVa)H,
NSPSA-EEEE-(Illb)OH, NSPSA-EEEEE-(Illb)OH, NSPSA-EEEEEE-(Illb)OH, NSPSA-EEEEEEE-(IIIb)OH, NSPSA-EEEEEEEE-(IIIb)OH, NSPSA-EEEE-(IIIb)NH₂, NSPSA-EEEEE-(IIIb)NH₂, NSPSA-EEEEEE-(IIIb)NH₂, NSPSA-EEEEEEE-(IIIb)NH₂, NSPSA-EEEEEEEE-(IIIb)NH₂, NSPSA-eeee-(IIIb)NH₂, NSPSA-eeeee-(IIIb)NH₂, NSPSA-eeeeee-(IIIb)NH₂, NSPSA-eeeeeee-(IIIb)NH₂, NSPSA-eeeeeeee-(IIIb)NH₂, NSPSA-eeeeeeeee-(IIIb)NH₂,
NSPSA-eeee-(Illb)OH, NSPSA-eeeee-(Illb)OH, NSPSA-eeeeee-(Illb)OH, NSPSA-eeeeeee-(Illb)OH, NSPSA-eeeeeeee-(Illb)OH, NSPSA-eeeeeeeee-(Illb)OH, NSP-DMAE-HEG-Glu-EEEE-(Illb)OH, NSP-DMAE-HEG-Glu-EEEEE-(Illb)OH, NSP-DMAE-HEG-Glu-EEEEEE-(Illb)OH, NSP-DMAE-HEG-Glu-EEEEEEE-(Illb)OH, NSP-DMAE-HEG-Glu-EEEEEEEE-(IIIb)OH, NSP-DMAE-HEG-Glu-EEEE-(IIIb)NH₂, NSP-DMAE-HEG-Glu-EEEEE-(IIIb)NH₂, NSP-DMAE-HEG-Glu-EEEEEE-(IIIb)NH₂, NSP-DMAE-HEG-Glu-EEEEEEE-(IIIb)NH₂, NSP-DMAE-HEG-Glu-EEEEEEEE-(IIIb)NH₂,
NSP-DMAE-HEG-Glu-eeee-(IIIb)NH₂, NSP-DMAE-HEG-Glu-eeeee-(IIIb)NH₂, NSP-DMAE-HEG-Glu-eeeeee-(IIIb)NH₂, NSP-DMAE-HEG-Glu-eeeeeee-(IIIb)NH₂, NSP-DMAE-HEG-Glu-eeeeeeee-(IIIb)NH₂, NSP-DMAE-HEG-Glu-eeeeeeeee-(IIIb)NH₂, NSP-DMAE-HEG-Glu-eeee-(Illb)OH, NSP-DMAE-HEG-Glu-eeeee-(Illb)OH, NSP-DMAE-HEG-Glu-eeeeee-(Illb)OH, NSP-DMAE-HEG-Glu-eeeeeee-(Illb)OH, NSP-DMAE-HEG-Glu-eeeeeeee-(Illb)OH, NSP-DMAE-HEG-Glu-eeeeeeeee-(Illb)OH,
NSP-DMAE-EEEE-(Illb)OH, NSP-DMAE-EEEEE-(Illb)OH, NSP-DMAE-EEEEEE-(IIIb)OH, NSP-DMAE-EEEEEEE-(Illb)OH, NSP-DMAE-EEEEEEEE-(Illb)OH, NSP-DMAE-EEEE-(Illb)NH2, NSP-DMAE-EEEEE-(Illb)NH2, NSP-DMAE-EEEEEE-(Illb)NH2, NSP-DMAE-EEEEEEE-(Illb)NH2, NSP-DMAE-EEEEEEEE-(Illb)NH2, NSP-DMAE-eeee-(Illb)NH2, NSP-DMAE-eeeee-(Illb)NH2, NSP-DMAE-eeeeee-(IIIb)NH₂, NSP-DMAE-eeeeeee-(Illb)NH2, NSP-DMAE-eeeeeeee-(Illb)NH2, NSP-DMAE-eeeeeeeee-(Illb)NH2,
NSP-DMAE-eeee-(Illb)OH, NSP-DMAE-eeeee-(Illb)OH, NSP-DMAE-eeeeee-(Illb)OH, NSP-DMAE-eeeeeee-(Illb)OH, NSP-DMAE-eeeeeeee-(Illb)OH, NSP-DMAE-eeeeeeeee-(IIIb)OH,
NSP-2,7-(OMHEG)2-DMAE-EEEE-(IIIb)OH, NSP-2,7-(OMHEG)2-DMAE-EEEEE-(IIIb)OH, NSP-2,7-(OMHEG)2-DMAE-EEEEEE-(Illb)OH, NSP-2,7-(OMHEG)2-DMAE-EEEEEEE-(Illb)OH, NSP-2,7-(OMHEG)2-DMAE-EEEEEEEE-(Illb)OH, NSP-2,7-(OMHEG)2-DMAE-EEEE-(IIIb)NH₂, NSP-2,7-(OMHEG)2-DMAE-EEEEE-(IIIb)NH₂, NSP-2,7-(OMHEG)2-DMAE-EEEEEE-(IIIb)NH₂, NSP-2,7-(OMHEG)2-DMAE-EEEEEEE-(IIIb)NH₂, NSP-2,7-(OMHEG)2-DMAE-EEEEEEEE-(IIIb)NH₂, NSP-2,7-(OMHEG)2-DMAE-eeee-(IIIb)NH₂, NSP-2,7-(OMHEG)2-DMAE-eeeee-(IIIb)NH₂, NSP-2,7-(OMHEG)2-DMAE-eeeeee-(IIIb)NH₂, NSP-2,7-(OMHEG)2-DMAE-eeeeeee-(IIIb)NH₂, NSP-2,7-(OMHEG)2-DMAE-eeeeeeee-(IIIb)NH₂, NSP-2,7-(OMHEG)2-DMAE-eeeeeeeee-(IIIb)NH₂,
NSP-2,7-(OMHEG)2-DMAE-eeee-(IIIb)OH, NSP-2,7-(OMHEG)2-DMAE-eeeee-(Illb)OH, NSP-2,7-(OMHEG)2-DMAE-eeeeee-(IIIb)OH, NSP-2,7-(OMHEG)2-DMAE-eeeeeee-(IIIb)OH, NSP-2,7-(OMHEG)2-DMAE-eeeeeeee-(IIIb)OH, NSP-2,7-(OMHEG)2-DMAE-eeeeeeeee-(Illb)OH,
NSPSA-EEEEK-(IVb)H, NSPSA-EEEEEK-(IVb)H, NSPSA-EEEEEEK-(IVb)H, NSPSA-EEEEEEEK-(IVb)H, NSPSA-EEEEEEEEK-(IVb)H, NSPSA-eeeeK-(IVb)C(O)CH3, NSPSA-eeeeeK-(IVb)C(O)CH3, NSPSA-eeeeeeK-(IVb)C(O)CH3, NSPSA-eeeeeeeK-(IVb)C(O)CH3, NSPSA-eeeeeeeeK-(IVb)C(O)CH3, NSPSA-eeeeeeeeeK-(IVb)C(O)CH3, NSPSA-eeeeK-(IVb)H, NSPSA-eeeeeK-(IVb)H, NSPSA-eeeeeeK-(IVb)H, NSPSA-eeeeeeeK-(IVb)H, NSPSA-eeeeeeeeK-(IVb)H, NSPSA-eeeeeeeeeK-(IVb)H,
NSP-DMAE-HEG-Glu-EEEEK-(IVb)H, NSP-DMAE-HEG-Glu-EEEEEK-(IVb)H, NSP-DMAE-HEG-Glu-EEEEEEK-(IVb)H, NSP-DMAE-HEG-Glu-EEEEEEEK-(IVb)H, NSP-DMAE-HEG-Glu-EEEEEEEEK-(IVb)H, NSP-DMAE-HEG-Glu-eeeeK-(IVb)C(O)CH3, NSP-DMAE-HEG-Glu-eeeeeK-(IVb)C(O)CH3, NSP-DMAE-HEG-Glu-eeeeeeK-(IVb)C(O)CH3, NSP-DMAE-HEG-Glu-eeeeeeeK-(IVb)C(O)CH3, NSP-DMAE-HEG-Glu-eeeeeeeeK-(IVb)C(O)CH3, NSP-DMAE-HEG-Glu-eeeeeeeeeK-(IVb)C(O)CH3,
NSP-DMAE-HEG-Glu-eeeeK-(IVb)H, NSP-DMAE-HEG-Glu-eeeeeK-(IVb)H, NSP-DMAE-HEG-Glu-eeeeeeK-(IVb)H, NSP-DMAE-HEG-Glu-eeeeeeeK-(IVb)H, NSP-DMAE-HEG-Glu-eeeeeeeeK-(IVb)H, NSP-DMAE-HEG-Glu-eeeeeeeeeK-(IVb)H,
NSP-DMAE-EEEEK-(IVb)H, NSP-DMAE-EEEEEK-(IVb)H, NSP-DMAE-EEEEEEK-(IVb)H, NSP-DMAE-EEEEEEEK-(IVb)H, NSP-DMAE-EEEEEEEEK-(IVb)H, NSP-DMAE-eeeeK-(IVb)C(O)CH3, NSP-DMAE-eeeeeK-(IVb)C(O)CH3, NSP-DMAE-eeeeeeK-(IVb)C(O)CH3, NSP-DMAE-eeeeeeeK-(IVb)C(O)CH3, NSP-DMAE-eeeeeeeeK-(IVb)C(O)CH3, NSP-DMAE-eeeeeeeeeK-(IVb)C(O)CH3,
NSP-DMAE-eeeeK-(IVb)H, NSP-DMAE-eeeeeK-(IVb)H, NSP-DMAE-eeeeeeK-(IVb)H, NSP-DMAE-eeeeeeeK-(IVb)H, NSP-DMAE-eeeeeeeeK-(IVb)H, NSP-DMAE-eeeeeeeeeK-(IVb)H,
NSP-2,7-(OMHEG)2-DMAE-EEEEK-(IVb)H, NSP-2,7-(OMHEG)2-DMAE-EEEEEK-(IVb)H, NSP-2,7-(OMHEG)2-DMAE-EEEEEEK-(IVb)H, NSP-2,7-(OMHEG)2-DMAE-EEEEEEEK-(IVb)H, NSP-2,7-(OMHEG)2-DMAE-EEEEEEEEK-(IVb)H, NSP-2,7-(OMHEG)2-DMAE-eeeeK-(IVb)C(O)CH3, NSP-2,7-(OMHEG)2-DMAE-eeeeeK-(IVb)C(O)CH3, NSP-2,7-(OMHEG)2-DMAE-eeeeeeK-(IVb)C(O)CH3, NSP-2,7-(OMHEG)2-DMAE-eeeeeeeK-(IVb)C(O)CH3, NSP-2,7-(OMHEG)2-DMAE-eeeeeeeeK-(IVb)C(O)CH3, NSP-2,7-(OMHEG)2-DMAE-eeeeeeeeeK-(IVb)C(O)CH3,
NSP-2,7-(OMHEG)2-DMAE-eeeeK-(IVb)H, NSP-2,7-(OMHEG)2-DMAE-eeeeeK-(IVb)H, NSP-2,7-(OMHEG)2-DMAE-eeeeeeK-(IVb)H, NSP-2,7-(OMHEG)2-DMAE-eeeeeeeK-(IVb)H, NSP-2,7-(OMHEG)2-DMAE-eeeeeeeeK-(IVb)H, NSP-2,7-(OMHEG)2-DMAE-eeeeeeeeeK-(IVb)H.

Die vorliegende Erfindung betrifft ein Verfahren zur quantitativen Bestimmung eines freien Thyroid-Hormons. Das Verfahren umfasst:
a. Bereitstellen einer Blutprobe (Humanserum, Humanplasma);
b. Kombinieren der Blutprobe mit einem Fängerreagenz in einem Reaktionsgefäß unter Erhalt eines ersten Gemisches, wobei das Fängerreagenz ein Magnetpartikel-Konjugat umfasst, und wobei das Magnetpartikel-Konjugat einen Magnetpartikel und einen spezifischen Binder des zu bestimmenden Thyroid-Hormons (entweder T3 oder T4) umfasst;
c. Aufarbeiten des ersten Gemisches;
d. Zugeben eines Detektorreagenzes in das Reaktionsgefäß unter Erhalt eines zweiten Gemisches, wobei das Detektorreagenz einen Thyronin-Derivat-Tracer der Formel (II) umfasst;
e. Aufarbeiten des zweiten Gemisches;
f. Auslösen der Chemilumineszenz des an das Magnetpartikel-Konjugat gebundenen Thyroid-Derivat-Tracers durch Zugabe mindestens eines Vorbereitungsreagenzes und mindestens eines Initiationsreagenzes;
g. Messen der Chemilumineszenz; und
h. quantitatives Bestimmen des freien Thyroid-Hormons.

Ein Thyronin-Derivat-Tracer der Formel (II) hat die Struktur

CL-Y-TX (II).

Darin steht
CL für eine Chemilumineszenzgruppe,
Y für ein lineares Peptid mit 3 bis 11 Aminosäuren, wobei mindestens die Hälfte der Aminosäuren des linearen Peptids saure Aminosäuren sind, und
TX für eine Gruppe der Formel (III) oder (IV)

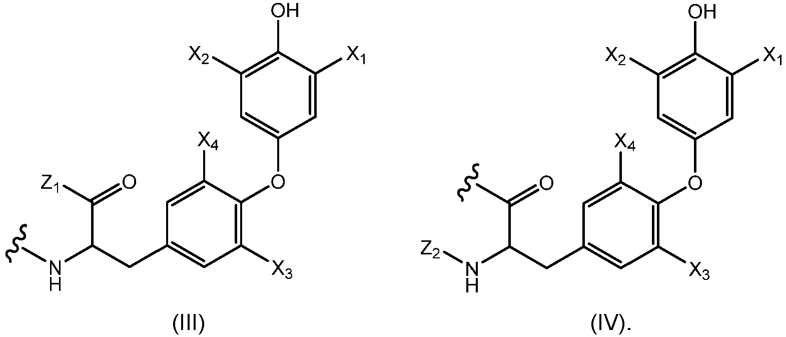

Darin stehen X₁, X₂, X₃ und X₄ unabhängig voneinander für I, H, Br, CI oder CN, Z₁ für OH oder NH₂ und Z₂ für H oder C(O)CH₃.

Das Verfahren gemäß der vorliegenden Erfindung ermöglicht es, ein freies Thyroid-Hormon, also fT3 oder fT4, quantitativ zu bestimmen. Das Verfahren verläuft als heterogener 2-Schritt-lmmunoassay.

### Schritt a.

Die Blutprobe ist bevorzugt Humanserum oder Humanplasma. Die Blutprobe kann eine zellfreie Probe sein, insbesondere eine zellfreie Serum- oder Plasmaproben. Zellfreie Serumproben können durch Abnahme einer Blutprobe, Initiierung der Gerinnung (z.B. durch Zugabe eines Gerinnungsaktivators oder Verwendung eines Blutröhrchens mit Gerinnungsaktivatoren), Inkubation, Zentrifugation und Abtrennen der flüssigen Phase erhalten werden. Plasmaproben können durch Abnahme einer Blutprobe, Verhindern der Gerinnung (z.B. durch Zugabe eines Antikoagulans wie EDTA oder Verwendung eines EDTA oder Heparin-Blutröhrchens), Zentrifugation und Abtrennen der flüssigen Phase erhalten werden.

### Schritt b.

Die Blutprobe wird mit einem Fängerreagenz in einem Reaktionsgefäß unter Erhalt eines ersten Gemisches kombiniert.

Das Fängerreagenz liegt bevorzugt in flüssiger Form vor. Verfahren zum Kombinieren zweier in flüssigem Medium vorliegender Komponenten sind dem Fachmann hinlänglich bekannt. Bevorzugt werden die beiden Komponenten nacheinander in ein Reaktionsgefäß pipettiert und anschließend gemischt, indem z.B. das Reaktionsgefäß durch Vortexen, Schwenken oder Schütteln bewegt wird oder das enthaltene Flüssigkeitsvolumen durch Rühren oder wiederholtes Aufsaugen und Abgeben vermengt wird.

Das Fängerreagenz umfasst ein Magnetpartikel-Konjugat. Das Magnetpartikel-Konjugat umfasst einen Magnetpartikel und einen spezifischen Binder des zu bestimmenden Thyroid-Hormons T3 oder T4. Durch das Kombinieren der Blutprobe mit dem Fängerreagenz bindet das zu bestimmende Thyroid-Hormon T3 oder T4 an den spezifischen Binder des Magnetpartikel-Konjugats.

Der spezifische Binder weist eine hohe Bindungsstärke zu dem zu bestimmenden Thyroid-Hormon - entweder T3 oder T4 - auf. Die Bindungsstärke wird als hoch bezeichnet, wenn die Affinitätskonstante des Binders zum zu bestimmenden Thyroid-Hormon größer oder gleich 1*10⁹ I/mol ist. Bevorzugt weist der spezifische Binder eine Affinitätskonstante zu dem zu bestimmenden Thyroid-Hormon von mindestens 1*10¹⁰ I/mol, weiter bevorzugt von mindestens 2*10¹⁰ I/mol auf.

Bevorzugt ist der spezifische Binder des zu bestimmenden Thyroid-Hormons T3 oder T4 aus der Gruppe bestehend aus Antikörpern, Antikörperfragmenten, Nanobodies, Rezeptoren und Aptameren ausgewählt. Besonders bevorzugt ist der spezifische Binder ein Antikörper.

Als Antikörper sind grundsätzlich monklonale und polyklonale Antikörper geeignet. Bevorzugt ist der Antikörper ein monklonaler Antikörper. Beispiele für geeignete Antikörper sind der von Meridian Bioscience vertriebene monoklonale Anti-T4-Antikörper MAT02-525 (Clone 204-14525), der von US Biological Life sciences vertriebene monoklonale Antikörper T5460-01G (Clone 9L720), beide Produkte verweisen in Ihren Datenblättern auf Bellisario, R., et al., (2000), Clinical Chemistry, 46 (9), 1422-1424. Beispiele für Antikörperfragmente sind z.B. F(ab')₂-Fragmente oder Fab-Fragmente.

Bevorzugt ist der spezifische Binder über einen Spacer mit dem Magnetpartikel gekoppelt.

Beispielsweise weist der spezifische Binder eine erste Bindungseinheit und der Magnetpartikel eine zweite Bindungseinheit auf, wobei die erste und die zweite Bindungseinheit aneinanderbinden können. Die Bindungseinheiten können kovalent oder nicht-kovalent aneinanderbinden, bevorzugt nicht-kovalent.

Der spezifische Binder kann über kovalente Bindungen, bevorzugt über einen Linker, an die erste Bindungseinheit gebunden sein. Bevorzugt ist der spezifische Binder über einen Linker kovalent an Biotin gebunden. Der Linker kann z.B. aus ein oder mehreren PEG-Einheiten bestehen und kann optional zusätzlich auch noch einen Farbstoff beinhalten, der nach Kopplung die Bestimmung der mittleren Anzahl der Linker pro Binder ermöglicht. Beispiele für geeignete Biotin-Linker-Farbstoff-Konjugate, die für die Ankoppelung an den Antikörper NHS-aktiviert sind, sind die Produkte Sulfo ChromaLink Biotin (Catalog# B-1007) sowie ChromaLink Biotin (Catalog# B-1001), wie beispielsweise durch die Firma Vector Laboratories angeboten.

Der Magnetpartikel kann mit der zweiten Bindungseinheit beschichtet sein. Bevorzugt weisen die Magnetpartikel eine mit Streptavidin beschichtete Oberfläche auf.

Der spezifische Binder ist bevorzugt über eine nicht-kovalente Biotin-Streptavidin-Bindung mit dem Magnetpartikel gekoppelt.

Die Magnetpartikel und die spezifischen Binder liegen im Magnetpartikel-Konjugat bevorzugt in einem Massenverhältnis von 25:1 bis 2500:1 vor. Zum Beispiel liegen die Magnetpartikel und die spezifischen Binder in einem Massenverhältnis von mindestens 30:1 oder mindestens 50:1 oder mindestens 75:1 oder mindestens 100:1 oder mindestens 125:1 vor. Zum Beispiel liegen die Magnetpartikel und die spezifischen Binder in einem Massenverhältnis von nicht mehr als 2000:1 oder nicht mehr als 1500:1 oder nicht mehr als 1000:1 oder nicht mehr als 500:1 oder nicht mehr als 400:1 vor. Besonders bevorzugt liegen die Magnetpartikel und die spezifischen Binder in einem Massenverhältnis von 150:1 bis 350:1 vor.

Die Magnetpartikel können eine Größe von 0,5 bis 8 µm, z.B. von 1 bis 5 µm, bevorzugt von 2 bis 3 µm aufweisen.

Die Magnetpartikel können eine beliebige Form aufweisen. Sie sind bevorzugt annähernd sphärisch.

Bevorzugt umfasst das Fängerreagenz einen Puffer. Geeignete Puffer sind beispielsweise phosphatgepufferte Salzlösungen (PBS-Puffer). Die Konzentration des Puffers kann im Bereich von 5 bis 200 mM liegen. Geeignete pH-Werte liegen beispielsweise in einem Bereich von 6,5 bis 8,2, bevorzugt von 6,8 bis 7,8, besonders bevorzugt von 7,0 bis 7,4.

Das Fängerreagenz kann zusätzlich ein Konservierungsmittel, wie z.B. Natriumazid, sowie ein Immunglobulin enthalten. Optional können auch ein oder mehrere Detergentien und oder ein oder mehrere Proteine, z.B. Albumin einhalten sein.

Bevorzugt umfasst das Fängerreagenz ein Albumin, z.B. Humanes Serumalbumin (HSA), Bovines Serumalbumin (BSA), rekombinantes Albumin, delipidiertes Albumin oder mit Aktivkohle aufgereinigtes Albumin, weiter bevorzugt rekombinantes Albumin (rHSA) oder mit Aktivkohle aufgereinigtes Albumin. Besonders bevorzugt umfasst das Detektorreagenz rekombinantes HSA (rHSA).

### Schritt c.

Das erste Gemisch wird aufgearbeitet. Das Aufarbeiten des ersten Gemisches umfasst zumindest
i. Inkubieren des ersten Gemisches;
ii. Immobilisieren des Magnetpartikel-Konjugates an der Wand des Reaktionsgefäßes;
iii. Entfernen der flüssigen Phase; und
iv. Waschen der festen Phase durch mehrmalige Abgabe und Aufnahme eines Waschpuffers;

Gemäß Schritt i. wird das erste Gemisch inkubiert. Bevorzugt wird das erste Gemisch bei 37°C inkubiert. Beim Inkubieren kann das erste Gemisch prinzipiell ruhen oder in Bewegung gehalten werden, beispielsweise gerührt, geschüttelt, geschwenkt oder gevortext werden. Rühren, Schütteln oder Vortexen kann durchgehend oder von Zeit zu Zeit erfolgen.

Bevorzugt beträgt die Inkubationszeit des ersten Gemisches 1 min bis 60 min, z.B. 5 bis 40 min oder 10 bis 30 min, besonders bevorzugt 15 bis 20min.

Die Inkubation kann prinzipiell unter verschiedenen Lichtbedingungen gleichermaßen erfolgen, beispielsweise unter Lichtausschluss, bei Raumlicht oder Bestrahlung. Bevorzugt erfolgt die Inkubation unter Lichtausschluss.

Gemäß Schritt ii. wird das Magnetpartikel-Konjugat an der Wand des Reaktionsgefäßes immobilisiert. Dies kann beispielsweise durch Anlegen eines magnetischen Feldes an die Wand des Reaktionsgefäßes erfolgen.

Gemäß Schritt iii. wird die flüssige Phase entfernt. Prinzipiell kann die flüssige Phase auf allen dem Fachmann bekannten Wegen entfernt werden, beispielsweise durch Pipettieren oder Absaugen. Bevorzugt wird die flüssige Phase mittels Absaugen entfernt.

Gemäß Schritt iv. wird die feste Phase durch mehrmalige Abgabe und Aufnahme eines Waschpuffers gewaschen. Beispielsweise wird 3x oder 4x oder 5x oder 6x gewaschen.

Der verwendete Waschpuffer ist bevorzugt geeignet, Komponenten zu entfernen, die nicht Teil des Magnetpartikel-Konjugats, an den optional zu bestimmendes Thyroid-Hormon T3 oder T4 gebunden ist, sind. Beispiele hierfür sind Komponenten aus der Probe inklusive der nicht an das Magnetpartikel-Konjugat gebundenen Thyroid-Hormone T3 und T4, insbesondere auch in Protein-gebundener Form, die in der Probe enthaltenen T4-bindenden Proteine und Komponenten des Fängerreagenzes, die nicht Teil des Magnetpartikelkonjugates sind.

Bevorzugt ist der Waschpuffer ein Phosphatpuffer oder ein PBS-Puffer. Der bevorzugte pH-Wert des Waschpuffers liegt im Bereich von 7,0 bis 7,5.

Bevorzugt umfasst der Waschpuffer ein Detergens. Das Detergens kann anionisch, kationisch, nicht-ionisch oder zwitterionisch sein. Detergenzien sind dem Fachmann hinläglich bekannt. Beispiele für anionische Detergenzien sind Alkylbenzolsulfonate mit einer Alkylgruppe mit 8 bis 24 C-Atomen, Alkylsulfonate mit einer Alkylgruppe mit 8 bis 24 C-Atomen und Alkalisalze von Fettsäuren. Beispiele für kationische Detergenzien sind Tetraalkylamooniumsalze mit mindestens einer Alkylgruppe mit 8 bis 24 C-Atomen (z.B. Distearyldimethylammoniumchlorid oder Cetyltrimethylammoniumbromid) und Esterquats basierend auf mit Fettsäure veresterten quartären Triethanol-Methyl-Ammonium- oder quartären Diethanol-Dimethyl-Ammonium-Verbindungen. Beispiele für nicht-ionische Detergenzien sind Ester oder Ether aus Polyoxyethylenen und aliphatischen Alkoholen oder Carbonsäuren, z.B. Polyoxyethylen-23-Laurylether. Beispiele für zwitterionische Detergenzien sind CHAPS (3-[(3-Cholamidopropyl)dimethylammonio]-1-propanesulfonat) und CHAPSO (3-[(3-Cholamidopropyl)dimethylammonio]-2-hydroxy-1-propanesulfonat). Besonders bevorzugt umfasst der Waschpuffer ein nicht-ionisches Detergens.

Der Waschpuffer kann zudem weitere Komponenten umfassen, beispielsweise Konservierungsstoffe.

Das Waschen der festen Phase gemäß Schritt iv. kann ein Resuspendieren und Reimmobilisieren der Partikel des Magnetpartikel-Konjugats im Waschpuffer umfassen. Dies kann beispielsweise
- durch Entfernen des magnetischen Feldes von der Wand des Reaktionsgefäßes,
- Durchmischen der festen Phase in dem Waschpuffen (z.B. durch Vortexen, Schütteln oder Schwenken) und
- erneutes Anlegen eines magnetischen Feldes an der Wand des Reaktionsgefäßes erfolgen.

### Schritt d.

Ein Detektorreagenz wird unter Erhalt eines zweiten Gemisches in das Reaktionsgefäß gegeben.

Das Detektorreagenz umfasst einen Thyronin-Derivat-Tracer der Formel (II). Die oben beschriebenen bevorzugten und beispielhaften Merkmale des Thyronin-Derivat-Tracers der Formel (II) gelten entsprechend für den in diesem Verfahren verwendeten Thyronin-Derivat-Tracer der Formel (II).

Bevorzugt stehen X₁, X₂, X₃ und X₄ unabhängig voneinander für I oder H. Bevorzugt steht mindestens eines von X₁, X₂, X₃ und X₄ für I, weiter bevorzugt stehen mindestens zwei von X₁, X₂, X₃ und X₄ für I. Besonders bevorzugt stehen X₁, X₃ und X₄ für I und X₂ für H.

Der Thyronin-Derivat-Tracer der Formel (II) kann in Abhängigkeit des zu bestimmenden Thyroid-Hormons ausgewählt werden, bevorzugt weist das Thyronin-Derivat des Thyronin-Derivat-Tracers einen lod-Substituenten weniger als das zu bestimmende Thyroid-Hormon auf. Beispielsweise wird zur Bestimmung von fT4 ein T3- oder rT3-basierter Tracer, also ein Tracer der TX-Gruppe einer der oben beschriebenen Formeln (!!!c), (IIId), (IVc) oder (IVd), und zur Bestimmung von fT3 ein T2-basierter Tracer, also ein Tracer der TX-Gruppe einer der oben beschriebenen Formeln (Illa), (IIIb) (IVa) oder (IVb), bevorzugt.

Bevorzugt umfasst das Detektorreagenz ein Albumin, z.B. Humanes Serumalbumin (HSA), Bovines Serumalbumin (BSA), rekombinantes Albumin oder mit Aktivkohle aufgereinigtes Albumin, weiter bevorzugt rekombinantes Albumin (rHSA) oder mit Aktivkohle aufgereinigtes Albumin. Besonders bevorzugt umfasst das Detektorreagenz rekombinantes HSA (rHSA).

Das Detektorreagenz enthält Albumin (bevorzugt rHSA) bevorzugt in einer Menge von 0,1 bis 5 Gew.-% besonders bevorzugt in einer Menge von 0,5 bis 2 Gew.-%, jeweils bezogen auf die Gesamtmasse des Detektorreagenzes.

Bevorzugt ist das im Detektorreagenz, im Fängerreagenz oder im Detektor- und Fängerreagenz enthaltene Albumin rekombinantes Albumin oder mit Aktivkohle aufgereinigtes Albumin.

Das Detektorreagenz liegt bevorzugt in flüssiger Form vor. Bevorzugt umfasst das Detektorreagenz einen Puffer. Geeignete Puffer sind beispielsweise phosphatgepufferte Salzlösungen (PBS-Puffer) oder Tris(hydroxymethyl)aminomethan-Puffer (TRIS-Puffer). Die Konzentration des Puffers kann im Bereich von 5 bis 200 mM liegen. Geeignete pH-Werte liegen beispielsweise in einem Bereich von 6,5 bis 9, bevorzugt von 6,8 bis 8,0, besonders bevorzugt von 7,0 bis 7,5.

Das Detektorreagenz enthält den Thyronin-Derivat-Tracer der Formel (II) bevorzugt in einer Konzentration von 0,1 bis 400 ng/mL, z.B. von 1 bis 200 ng/mL oder von 5 bis 100 ng/mL oder von 10 bis 70 ng/mL oder von 12 bis 50 ng/mL, besonders bevorzugt von 15-25 ng/mL.

Das Detektorreagenz enthält bevorzugt mindestens ein Additiv. Beispiele für geeignete Additive sind Stabilisatoren, Antioxidanzien, Detergenzien, Mono- oder Disaccharide oder Kombinationen davon. Bevorzugt umfasst das Detektorreagenz einen Stabilisator, besonders bevorzugt EDTA. Bevorzugt umfasst das Detektorreagenz ein Antioxidans, besonders bevorzugt 4-Methoxyphenol. Bevorzugt umfasst das Detektorreagenz einen Stabilisator und ein Antioxidans, besonders bevorzugt EDTA und 4-Methoxyphenol. Das Detektorreagenz kann zusätzlich ein Konservierungsmittel, wie z.B. Natriumazid, sowie ein Immunglobulin enthalten.

Bevorzugt wird das Detektorreagenz als 2-Komponenten-System bereitgestellt. In einem solchen 2-Komponenten-System liegen zumindest der Thyronin-Derivat-Tracer und das Albumin vor Zugabe des Detektorreagenzes in Schritt d. räumlich voneinander getrennt von

Der Thyronin-Derivat-Tracer und das Albumin können jeweils in Puffer bereitgestellt werden. Zum Beispiel können der Thyronin-Derivat-Tracer und das Albumin in Puffern verschiedener pH-Werte bereitgestellt werden.

Der Thyronin-Derivat-Tracer kann in diesem Fall in einem Puffer mit leicht saurem pH-Wert bereitgestellt werden. Der Thyronin-Derivat-Tracer zeigt in leicht saurem Medium eine höhere Stabilität als in basischem Medium.

Das Albumin kann in diesem Fall in einem Puffer mit neutralem oder leicht basischem pH-Wert bereitgestellt werden. Bei Kombination der Komponenten wird bevorzugt ein pH-Wert im Bereich von 7,0 bis 8,5, z.B. von 7,0 bis 8,0 erreicht. Eine der beiden Komponenten kann eine höhere Pufferkapazität aufweisen, so dass diese nach Mischung der Komponenten maßgeblich den resultierenden pH-Wert beeinflusst.

### Schritt e.

Das zweite Gemisch wird aufgearbeitet. Das Aufarbeiten des zweiten Gemisches umfasst zumindest
i) Resuspendieren der Partikel des Magnetpartikel-Konjugat im Detektorreagenz;
ii) Inkubieren des zweiten Gemisches;
iii) Immobilisieren des Magnetpartikel-Konjugats an der Wand des Reaktionsgefäßes;
iv) Entfernen der flüssigen Phase; und
v) Waschen der festen Phase durch mehrmalige Abgabe und Aufnahme eines Waschpuffers;

Gemäß Schritt i) werden die Partikel des Magnetpartikel-Konjugats im Detektorreagenz resuspendiert. Dies kann durch Entfernen des magnetischen Feldes von der Wand des Reaktionsgefäßes und anschließendes Durchmischen der Komponenten im Reaktionsgefäß erfolgen. Das Durchmischen kann beispielsweise durch Vortexen, Schwenken oder Schütteln des Reaktiosgefäßes, durch Rühren oder durch wiederholtes Aufsaugen und Abgeben des zweiten Gemisches erfolgen. Rühren, Schütteln, Schwenken oder Vortexen kann durchgehend oder von Zeit zu Zeit erfolgen.

Gemäß Schritt ii) wird das zweite Gemisch inkubiert. Bevorzugt wird das zweite Gemisch bei 37 °C inkubiert. Beim Inkubieren kann das zweite Gemisch prinzipiell ruhen oder in Bewegung gehalten werden, beispielsweise gerührt, geschüttelt, gevortext oder geschwenkt werden. Rühren, Schütteln, Schwenken oder Vortexen kann durchgehend oder von Zeit zu Zeit erfolgen.

Bevorzugt beträgt die Inkubationszeit des zweiten Gemisches 1 min bis 60 min, z.B. 2 bis 20 min oder 2 bis 10 min, besonders bevorzugt 3 bis 5min.

Die Inkubation kann prinzipiell unter verschiedenen Lichtbedingungen gleichermaßen erfolgen, beispielsweise unter Lichtausschluss, bei Raumlicht oder Bestrahlung. Bevorzugt erfolgt die Inkubation unter Lichtausschluss.

Gemäß Schritt iii) wird des Magnetpartikel-Konjugat an der Wand des Reaktionsgefäßes immobilisiert. Dies kann beispielsweise durch Anlegen eines magnetischen Feldes an die Wand des Reaktionsgefäßes erfolgen.

Gemäß Schritt iv) wird die flüssige Phase entfernt. Prinzipiell kann die flüssige Phase auf allen dem Fachmann bekannten Wegen entfernt werden, beispielsweise durch Pipettieren oder Absaugen. Bevorzugt wird die flüssige Phase mittels Absaugen entfernt.

Gemäß Schritt v) wird die feste Phase durch mehrmalige Abgabe und Aufnahme eines Waschpuffers gewaschen. Beispielsweise wird 3x oder 4x oder 5 x oder 6x gewaschen.

Der verwendete Waschpuffer ist bevorzugt geeignet, Komponenten zu entfernen, die nicht Teil des Magnetpartikel-Konjugats, an den das zu bestimmende Thyroid-Hormon T3 oder T4 und der Thyronin-Derivat-Tracer gebunden ist, sind. Beispiele hierfür sind ungebundene Komponenten des Detektorreagenzes, insbesondere ungebundener Thyronin-Derivat-Tracer.

Bevorzugt ist der Waschpuffer ein Phosphatpuffer oder ein PBS-Puffer. Der bevorzugte pH-Wert des Waschpuffers liegt im Bereich von 7,0 bis 7,5.

Bevorzugt umfasst der Waschpuffer ein Detergens. Das Detergens kann anionisch, kationisch, nicht-ionisch oder zwitterionisch sein. Detergenzien sind dem Fachmann hinläglich bekannt. Beispiele für anionische Detergenzien sind Alkylbenzolsulfonate mit einer Alkylgruppe mit 8 bis 24 C-Atomen, Alkylsulfonate mit einer Alkylgruppe mit 8 bis 24 C-Atomen und Alkalisalze von Fettsäuren. Beispiele für kationische Detergenzien sind Tetraalkylamooniumsalze mit mindestens einer Alkylgruppe mit 8 bis 24 C-Atomen (z.B. Distearyldimethylammoniumchlorid oder Cetyltrimethylammoniumbromid) und Esterquats basierend auf mit Fettsäure veresterten quartären Triethanol-Methyl-Ammonium- oder quartären Diethanol-Dimethyl-Ammonium-Verbindungen. Beispiele für nicht-ionische Detergenzien sind Ester oder Ether aus Polyoxyethylenen und aliphatischen Alkoholen oder Carbonsäuren, z.B. Polyoxyethylen-23-Laurylether. Beispiele für zwitterionische Detergenzien sind CHAPS (3-[(3-Cholamidopropyl)dimethylammonio]-1-propanesulfonat) und CHAPSO (3-[(3-Cholamidopropyl)dimethylammonio]-2-hydroxy-1-propanesulfonat). Besonders bevorzugt umfasst der Waschpuffer ein nicht-ionisches Detergens.

Das Waschen der festen Phase gemäß Schritt v) kann ein Resuspendieren und Reimmobilisieren der Partikel des Magnetpartikel-Konjugats im Waschpuffer umfassen. Dies kann beispielsweise durch
- Entfernen des magnetischen Feldes von der Wand des Reaktionsgefäßes,
- Durchmischen der festen Phase in dem Waschpuffer (z.B. durch Vortexen, Schütteln oder Schwenken oder durch Aufwirbeln bei Abgabe des Flüssigkeitsvolumens) und
- erneutes Anlegen eines magnetischen Feldes an der Wand des Reaktionsgefäßes erfolgen.

### Schritt f.

Die Bestimmung des freien Thyroid-Hormons in der Probe erfolgt durch Bestimmen der Chemilumineszenz des an das Magnetpartikel-Konjugat gebundenen Thyroid-Derivat-Tracers. Die Chemilumineszenz wird durch Zugabe mindestens eines Vorbereitungsreagenzes und mindestens eines Initiationsreagenzes zu der in Schritt e. erhaltenen festen Phase ausgelöst.

Bevorzugt umfasst das Vorbereitungsreagenz eine wässrige Lösung umfassend eine Säure (z.B. HNO₃) und H₂O₂. Bevorzugt liegt H₂O₂ im deutlichen molaren Überschuss zum eingesetzten Thyronin-Derivat-Tracer vor.

Bevorzugt umfasst das Initiationsreagenz eine wässrige Lösung umfassend eine Base (z.B. NaOH) und ein Detergens. Als Detergenzien eignen sich beispielsweise die im Rahmen des Waschpuffers beschriebenen Detergenzien. Bevorzugt liegt das Initiationsreagenz ebenfalls im deutlichen molaren Überschuss zum eingesetzten Thyronin-Derivat-Tracer vor. Bevorzugt liegt die Menge an Base des Initiationsreagenzes in deutlichem molaren Überschuss im Vergleich zur Menge an Säure des Vorbereitungsreagenzes vor. Bevorzugt liegt der pH-Wert nach Zugabe des Initiationsreagenzes im stark basischen, z.B. bei mehr als pH 10 oder bei mehr als pH 11, besonders bevorzugt in einem pH-Bereich von 12 bis 13.

Mechanistisch kann das Auslösen der Chemilumineszenz beispielhaft wie in Natrajan (Org. Biomol. Chem. 12 (2014) 3887-3901) für NSP-haltige Acridiniumester dargestellt werden:

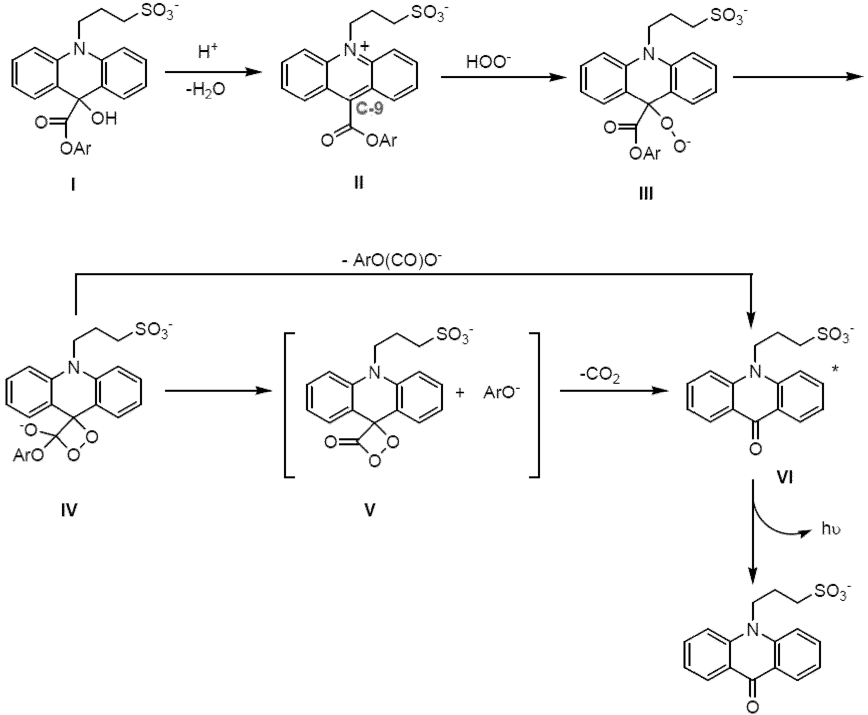

Die Anwesenheit eines Detergens kann die Signalstärke erhöhen und die Reaktion beschleunigen.

Bevorzugt umfasst das Auslösen der Chemilumineszenz:
a. das Zugeben des Vorbereitungsreagenzes,
b. das Durchmischen unter Resuspendieren des Magnetpartikel-Konjugats im Vorbereitungsreagenz und
c. das Zugeben des Initiationsreagenzes.

Beispielsweise kann das Durchmischen unter Resuspendieren des Magnetpartikel-Konjugats im Vorbereitungsreagenz erfolgen durch
- Entfernen des magnetischen Feldes von der Wand des Reaktionsgefäßes und
- Durchmischen des Magnetpartikel-Konjugats in dem Vorbereitungsreagenz (z.B. durch schnelles Zugeben des Vorbereitungsreagenzes, Vortexen, Schütteln, Schwenken oder mehrmaliges Aufnehmen und Abgeben mit einem Pipettor).

Das Durchmischen erfolgt bevorzugt über einen Zeitraum von 0,1 s bis 20 s, z.B. mehr als 0,1 s oder mehr als 0,5 s oder mehr als 1 s, z.B. bis zu 20 s oder bis zu 10 s oder bis zu 5 s oder bis zu 3 s.

Es ist anzunehmen, dass sich die Bindung zwischen Thyronin-Derivat-Tracer und Magnetpartikel-Konjugat im sauren Vorbereitungsreagenz-Milieu löst, so dass der Thyronin-Derivat-Tracer nicht mehr am Magnetpartikel gebunden ist. Optional können die Magnetpartikel durch Anlegen eines Magnetischen Feldes wieder an der Wand des Reaktionsgefäßes immobilisiert werden. Dadurch dass die Magnetpartikel aus dem Lichtweg zum Detektor entfernt sind, kann die Signalstärke der Chemilumineszenz erhöht werden.

Die Lösung des Initiationsreagenzes wird bevorzugt möglichst schnell zugegeben, beispielsweise in einem schnellen Strahl, um eine schnelle Durchmischung der Komponenen zu gewährleisten.

### Schritt g.

Die Chemilumineszenz wird mittels eines Lichtdetektors, bevorzugt mittels eines Photomultipliers, gemessen.

Die Chemilumineszenz wird bevorzugt für einen zeitlich begrenzten Zeitraum, der unmittelbar nach Zugabe des Initiationsreagenzes beginnt, gemessen. Die Chemilumineszenz kann innerhalb von 20 s, z.B. innerhalb von 10 s, bevorzugt innerhalb von 5 s nach Zugabe des Initiationsreagenzes gemessen werden.

### Schritt h.

Bevorzugt erfolgt die Quantifizierung des freien Thyroid-Hormons anhand eines Vergleichs mit einer Kalibrationskurve.

Zur Bestimmung der Kalibrationskurve werden mehrere Kalibratorproben, die durch ihre unterschiedlich hohen freien Thyroid-Hormon-Konzentrationen unterschiedlich hohe Chemilumineszenzsignale im beschriebenen Verfahren erzeugen, gemessen.

Die Bestimmung der Referenzkonzentrationen der Kalibratorlösungen gestaltet sich für freie Hormone komplizierter, als für viele andere Analyten, da die freie Hormonkonzentration sowohl von der im Blut enthaltenen Thyroid-Hormon-Konzentration als auch von der Konzentration der einzelnen T4-bindenden Proteine sowie möglichen weiteren Faktoren, wie z.B. dem Vorhandenseien von anderen Substanzen, die mit den Bindungsstellen der bindenden Proteine interagieren, abhängt. Das IFCC-Committee zur Standardisierung von Thyroid-Funktionstests (C-STFT) beschäftigt sich unter anderem mit diesem Problem und zeigt hier Lösungswege auf (https://www.ifcc-cstft.org/, De Grande, Clinical Chemistry 63(10) (2017) 1642-1652). Zudem verhalten sich kostengünstige Kalibrationsmatrices für den klinischen Gebrauch nicht immer genauso, wie klinische Proben. Daher muss der Hersteller den Kalibratoren Referenzwerte zuordnen, die für seinen Assay dazu führen, dass an der Kalibrierkurve abgelesene klinische Proben zu richtigen klinischen, im Idealfall standardisierten, Ergebnissen führen.

Typischerweise werden für die Kalibration des Assays bis zu 10 Kalibratoren, bevorzugt 2-6 Kalibratoren, weiter bevorzugt 2 oder 6 Kalibratoren, im Messbereich des freien Thyroid-Assays gemessen. Die Kalibratoren weisen jeweils unterschiedliche, zugeordnete freie Thyroid-Konzentrationen verteilt über den Messbereich des Assays auf.

Ein typischer Kalibrationsbereich für fT4 ist z.B. 0-100 pmol/l fT4. Ein typischer Kalibrationsbereich für fT3 ist z.B. 0-50 pmol/l fT3. Der untere Rand des Kalibrationsbereiches kann jedoch auch höher sowie der obere Rand des Kalibrationsbereiches höher oder niedriger liegen.

Nach Messung der Kalibratoren mit dem freien Thyroid-Assay können die Wertepaare der zugeordneten Konzentrationen und des Assaysignals graphisch aufgetragen werden und eine Kurve über alle Kalibratoren gefittet werden. Typischerweise kommen für den nicht-linearen Fit 4PL-Kurven (Four Parameter Logistic Regression) zum Einsatz. Da von einem nicht-linearen Zusammenhang ausgegangen werden muss, muss zudem bei Verwendung von nur 2 oder 3 Kalibratoren die typische Kalibrationsfunktion bekannt sein und wird dann anhand der 2-3 gemessenen Wertepaare skaliert.

Zur quantitativen Bestimmung des entsprechenden freien Thyroid-Hormons in einer Probe kann die gemessene Chemilumineszenz der Probe anhand einer so bestimmten Kalibrationskurve in die entsprechende freie Thyroid-Hormon-Konzentration quantifiziert werden.

Bevorzugt umfasst daher das quantitative Bestimmen des freien Thyroid-Hormons
(i) das Bestimmen der Chemilumineszenz in zwei bis 10 Kalibratorlösungen, wobei die Kalibratorlösungen das zu bestimmende Thyroid-Hormon in unterschiedlichen Konzentrationen enthalten und mindestens ein T4-bindendes Protein und wobei den Kalibratorlösungen, beispielsweise wie oben beschrieben, ein passender Referenzwert für das freie zu bestimmende Thyroid-Hormon für diesen Assay zugeordnet wurde;
(ii) Erstellen einer Kalibrationskurve anhand der zugeordneten freien Hormon-Konzentrationen der Kalibratorlösungen und der bestimmten Chemilumineszenz; und
(iii) Quantifizieren des Thyroid-Hormons mittels Vergleich der gemäß Schritt g. gemessenen Chemilumineszenz einer Blutprobe und der unter (ii) erstellten Kalibrationskurve.

Das hier beschriebene Verfahren oder einer oder mehrere der Schritte a. bis h. davon können teilautomatisiert oder vollautomatisiert durchgeführt werden.

Die vorliegende Erfindung betrifft auch ein Kit für einen heterogenen Immunoassay zur Bestimmung eines freien Thyroid-Hormons. Das Kit umfasst
i. ein Fängerreagenz umfassend ein Magnetpartikel-Konjugat, wobei das Magnetpartikel-Konjugat einen Magnetpartikel und einen spezifischen Binder des entsprechenden Thyroid-Hormones umfasst; und
ii. ein Detektorreagenz umfassend einen Thyronin-Derivat-Tracer der Formel (II) wie vorstehend beschrieben.

Die im Rahmen des Verfahrens beschriebenen bevorzugten und beispielhaften Ausführungsformen des Fänger- und Detektorreagenzes gelten entsprechend auch für das im Kit enthaltene Fängerreagenz und Detektorreagenz.

Die oben beschriebenen bevorzugten und beispielhaften Merkmale des Thyronin-Derivat-Tracers der Formel (II) gelten entsprechend für den in diesem Verfahren verwendeten Thyronin-Derivat-Tracer der Formel (II).

Bevorzugt stehen X₁, X₂, X₃ und X₄ unabhängig voneinander für I oder H. Bevorzugt steht mindestens eines von X₁, X₂, X₃ und X₄ für I, weiter bevorzugt stehen mindestens zwei von X₁, X₂, X₃ und X₄ für I. Besonders bevorzugt stehen X₁, X₃ und X₄ für I und X₂ für H.

Bevorzugt ist das Detektorreagenz ein 2-Komponenten-System wie im Rahmen des Verfahrens beschrieben.

Bevorzugt umfasst das Kit ferner einen Waschpuffer. Bevorzugt umfasst der Waschpuffer ein Detergens. Die im Rahmen des Verfahrens beschriebenen bevorzugten und beispielhaften Ausführungsformen des Waschpuffers gelten entsprechend auch für den im Kit enthaltenen Waschpuffer.

Bevorzugt umfasst das Kit zudem eine Kalibrationskomponente. Mit Hilfe der Kalibrationskomponente kann eine Kalibrationskurve erstellt werden. Die Kalibrationskomponente enthält Kalibratorstufen, die das zu bestimmende Thyroid-Hormon in verschiedenen Konzentrationen enthalten. Den Kalibratorstufen der Kalibrationskomponente ist eine freie Thyroid-Hormonkonzentration des zu bestimmenden Hormons zugeordnet. Die Kalibrationskomponente kann in flüssiger oder lyophillisierter Form bereitgestellt werden. Die Kalibratorstufen der Kalibrationskomponente enthalten bevorzugt eine Kalibrationsflüssigkeit, in der mindestens das zu bestimmende Thyroid-Hormon in verschiedenen Konzentrationen in einem flüssigen Medium, z.B. einer Serum-basierten Matrix oder einem wässrigen Puffer, der mindestens eins der T4-bindenden Proteine enthält, vorliegt. Liegt die Kalibrationskomponente in lyophillisierter Form vor, so muss diese vor Gebrauch durch ein geeignetes wäßriges Medium, z.B. Wasser, resuspendiert werden.

Bevorzugt liegen die Kalibrationslösungen mit den unterschiedlichen freien Thyroid-Konzentrationen in gebrauchsfertiger Form vor.

### Beispielhafte Ausführungsformen

1. Thyronin-Derivat-Tracer der Formel (II)

   CL-Y-TX (II)

   wobei
   CL für eine Chemilumineszenzgruppe steht,
   Y für ein lineares Peptid bestehend aus 3 bis 12 Aminosäuren steht, wobei mindestens die Hälfte der Aminosäuren des linearen Peptids saure Aminosäuren sind, und
   TX für eine Gruppe der Formel (III) oder (IV) 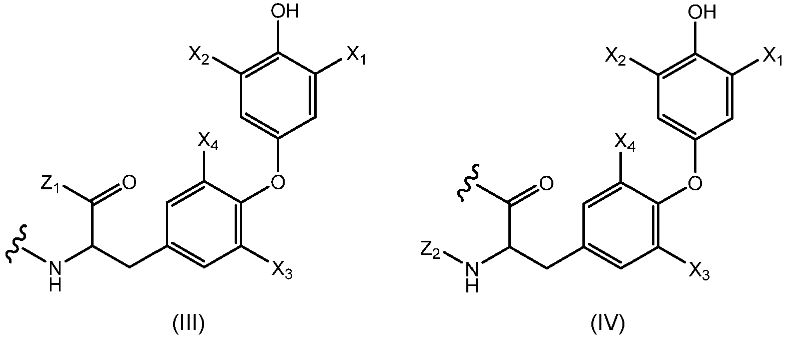
   steht,
      wobei X₁, X₂, X₃ und X₄ unabhängig voneinander für I, H, Br, CI oder CN stehen, Z₁ für OH oder NH₂ steht und
      Z₂ für H oder C(O)CH₃ steht.
2. Der Thyronin-Derivat-Tracer gemäß Ausführungsform 1, wobei die Aminosäuren D-Aminosäuren sind.
3. Der Thyronin-Derivat-Tracer gemäß einer der vorstehenden Ausführungsformen, wobei das lineare Peptid 5 bis 8 Aminosäuren umfasst.
4. Der Thyronin-Derivat-Tracer gemäß einer der vorstehenden Ausführungsformen, wobei mindestens die Hälfte der Aminosäuren des linearen Peptids Glutaminsäuren oder Asparaginsäuren sind.
5. Der Thyronin-Derivat-Tracer gemäß einer der vorstehenden Ausführungsformen, wobei das lineare Peptid 1 bis 6 Lysine umfasst.
6. Der Thyronin-Derivat-Tracer gemäß einer der vorstehenden Ausführungsformen, wobei X₁, X₂, X₃ und X₄ unabhängig voneinander für I oder H stehen.
7. Der Thyronin-Derivat-Tracer gemäß einer der vorstehenden Ausführungsformen, wobei mindestens eines von X₁, X₂, X₃ und X₄ für I steht, bevorzugt mindestens zwei von X₁, X₂, X₃ und X₄ für I stehen.
8. Der Thyronin-Derivat-Tracer gemäß einer der vorstehenden Ausführungsformen, wobei X₁, X₃ und X₄ für I stehen und X₂ für H steht.
9. Der Thyronin-Derivat-Tracer gemäß einer der vorstehenden Ausführungsformen, wobei Z₁ für NH₂ steht.
10. Der Thyronin-Derivat-Tracer gemäß einer der vorstehenden Ausführungsformen, wobei CL eine Chemilumineszenzgruppe basierend auf einem Chemilumineszenzfarbstoff ausgewählt aus der Gruppe bestehend aus Acridiniumester-Farbstoffen und Acridiniumsulfonamid-Farbstoffen ist.
11. Der Thyronin-Derivat-Tracer gemäß einer der vorstehenden Ausführungsformen, wobei CL und die N-terminale Aminosäure des linearen Peptids Y durch eine Peptidbindung verküpft sind, und wobei TX und die C-terminale Aminosäure des linearen Peptids Y durch eine Peptidbindung verküpft sind.
12. Der Thyronin-Derivat-Tracer gemäß einer der vorstehenden Ausführungsformen, wobei die C-terminale Aminosäure des linearen Peptids Y über die C-terminale Carboxygruppe oder über eine ε-NH₂-Gruppe mittels Peptidbindung mit TX verbunden ist.
13. Verfahren zur quantitativen Bestimmung eines freien Thyroid-Hormons umfassend
   a. Bereitstellen einer Blutprobe;
   b. Kombinieren der Blutprobe mit einem Fängerreagenz in einem Reaktionsgefäß unter Erhalt eines ersten Gemisches,
      wobei das Fängerreagenz ein Magnetpartikel-Konjugat umfasst, und
      wobei das Magnetpartikel-Konjugat einen Magnetpartikel und einen spezifischen Binder des zu bestimmenden Thyroid-Hormons umfasst;
   c. Aufarbeiten des ersten Gemisches zumindest umfassend
      i. Inkubieren des ersten Gemisches;
      ii. Immobilisieren des Magnetpartikel-Konjugats an der Wand des Reaktionsgefäßes;
      iii. Entfernen der flüssigen Phase; und
      iv. Waschen der festen Phase durch mehrmalige Abgabe und Aufnahme eines Waschpuffers;
   d. Zugeben eines Detektorreagenzes in das Reaktionsgefäß unter Erhalt eines zweiten Gemisches,
      wobei das Detektorreagenz einen Thyronin-Derivat-Tracer der Formel (II)

         CL-Y-TX (II)
      umfasst, wobei
         CL für eine Chemilumineszenzgruppe steht,
         Y für ein lineares Peptid bestehend aus 3 bis 12 Aminosäuren steht, wobei mindestens die Hälfte der Aminosäuren des linearen Peptids saure Aminosäuren sind, und
         TX für eine Gruppe der Formel (III) oder (IV) 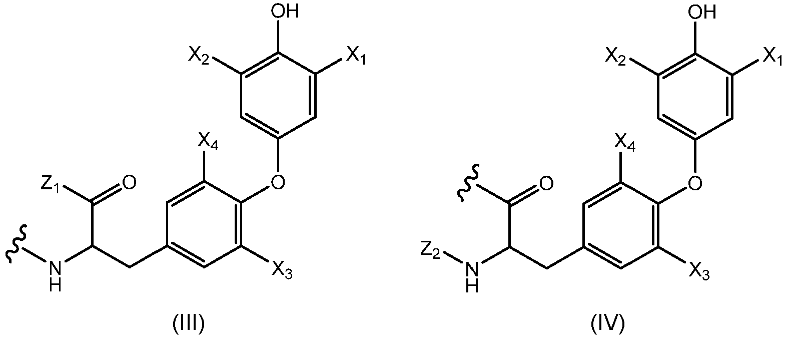
         steht,
            wobei X₁, X₂, X₃ und X₄ unabhängig voneinander für I, H, Br, CI oder CN stehen,
            Z₁ für OH oder NH₂ steht und
            Z₂ für H oder C(O)CH₃ steht;
   e. Aufarbeiten des zweiten Gemisches zumindest umfassend
      i) Resuspendieren der Partikel des Magnetpartikel-Konjugats im Detektorreagenz;
      ii) Inkubieren des zweiten Gemisches;
      iii) Immobilisieren des Magnetpartikel-Konjugats an der Wand des Reaktionsgefäßes;
      iv) Entfernen der flüssigen Phase; und
      v) Waschen der festen Phase durch mehrmalige Abgabe und Aufnahme eines Waschpuffers;
   f. Auslösen der Chemilumineszenz des an das Magnetpartikel-Konjugat gebundenen Thyroid-Derivat-Tracers durch Zugabe mindestens eines Vorbereitungsreagenzes und mindestens eines Initiationsreagenzes;
   g. Messen der Chemilumineszenz; und
   h. quantitatives Bestimmen des freien Thyroid-Hormons.
14. Das Verfahren gemäß Ausführungsform 13, wobei der Thyronin-Derivat-Tracer ein Thyronin-Derivat-Tracer gemäß einer der Ausführungsformen 1 bis 12 ist.
15. Das Verfahren gemäß einer der Ausführungsformen 13 oder 14, wobei X₁, X₂, X₃ und X₄ unabhängig voneinander für I oder H stehen.
16. Das Verfahren gemäß einer der Ausführungsformen 13 bis 15, wobei mindestens eines von X₁, X₂, X₃ und X₄ für I steht, bevorzugt mindestens zwei von X₁, X₂, X₃ und X₄ für I stehen.
17. Das Verfahren gemäß einer der Ausführungsformen 13 bis 16, wobei das Detektorreagenz ein Albumin, bevorzugt rekombinantes Albumin oder mit Aktivkohle aufgereinigtes Albumin, umfasst.
18. Das Verfahren gemäß Ausführungsform 17, wobei das Detektorreagenz ein Albumin in einer Menge von 0,1 bis 5 %, bezogen auf die Gesamtmasse Detektorreagenz, umfasst.
19. Das Verfahren gemäß einer der Ausführungsformen 13 bis 18, wobei der spezifische Binder des zu bestimmenden Thyroid-Hormons aus der Gruppe bestehend aus einem Antikörper, einem Antikörperfragment, einem Nanobody, einem Rezeptor und einem Aptamer ausgewählt ist.
20. Das Verfahren gemäß einer der Ausführungsformen 13 bis 19, wobei das Detektorreagenz als 2-Komponenten-System bereitgestellt wird.
21. Das Verfahren gemäß einer der Ausführungsformen 13 bis 20, wobei der spezifische Binder ein monoklonaler Antikörper ist.
22. Das Verfahren gemäß einer der Ausführungsformen 13 bis 21, wobei der spezifische Binder über einen Spacer mit dem Magnetpartikel gekoppelt ist.
23. Das Verfahren gemäß Ausführungsform 22, wobei der spezifische Binder über eine nicht-kovalente Biotin-Streptavidin-Bindung mit dem Magnetpartikel gekoppelt ist.
24. Das Verfahren gemäß einer der Ausführungsformen 13 bis 23, wobei die Magnetpartikel eine mit Streptavidin beschichtete Oberfläche aufweisen.
25. Das Verfahren gemäß einer der Ausführungsformen 13 bis 24, wobei der spezifische Binder kovalent über einen Linker, bevorzugt aus PEG-Einheiten , optional ferner umfassend einen Farbstoff zur Bestimmung des Anbindungsverhältnisses des Linkers zum Antikörper, an Biotin gebunden ist.
26. Das Verfahren gemäß einer der Ausführungsformen 13 bis 25, wobei die Magnetpartikel und die spezifischen Binder in einem Massenverhältnis von 25:1 bis 2500:1 im Magnetpartikel-Konjugat vorliegen.
27. Das Verfahren gemäß einer der Ausführungsformen 13 bis 26, wobei die Magnetpartikel annähernd sphärisch sind.
28. Das Verfahren gemäß einer der Ausführungsformen 13 bis 27, wobei die Magnetpartikel eine Größe von 0,5 bis 8 µm aufweisen.
29. Das Verfahren gemäß einer der Ausführungsformen 13 bis 28, wobei das Fängerreagenz ein Albumin, bevorzugt rekombinantes Albumin oder mit Aktivkohle aufgereinigtes Albumin, umfasst.
30. Das Verfahren gemäß Ausführungsform 29, wobei das Fängerreagenz 0,1 bis 5 Gew.-% Albumin, bezogen auf die Gesamtmasse Fängerreagenz, umfasst.
31. Das Verfahren gemäß einer der Ausführungsformen 13 bis 30, wobei das Fängerreagenz einen Puffer umfasst.
32. Das Verfahren gemäß einer der Ausführungsformen 13 bis 31, wobei das Fängerreagenz einen pH-Wert im Bereich von 6,5 bis 8,2 aufweist.
33. Das Verfahren gemäß einer der Ausführungsformen 13 bis 32, wobei das Detektorreagenz einen Puffer umfasst.
34. Das Verfahren gemäß einer der Ausführungsformen 13 bis 33, wobei der Thyronin-Derivat-Tracer im Detektorreagenz in einer Konzentration von 0,1 bis 400 ng/mL enthalten ist.
35. Das Verfahren gemäß einer der Ausführungsformen 13 bis 34, wobei das Detektorreagenz mindestens ein Additiv umfasst.
36. Das Verfahren gemäß einer der Ausführungsformen 13 bis 35, wobei die Inkubationszeit gemäß Schritt i. 1 min bis 60 min beträgt.
37. Das Verfahren gemäß einer der Ausführungsformen 13 bis 36, wobei die Inkubationszeit gemäß Schritt ii) 30 s bis 10 min beträgt
38. Das Verfahren gemäß einer der Ausführungsformen 13 bis 37, wobei die flüssige Phase gemäß Schritt iii. oder iv) oder beiden mittels Pipettieren oder mittels Absaugen entfernt wird.
39. Das Verfahren gemäß einer der Ausführungsformen 13 bis 38, wobei der Waschpuffer gemäß Schritt iv. oder v) oder beiden ein Detergens umfasst.
40. Das Verfahren gemäß einer der Ausführungsformen 13 bis 39, wobei das Waschen der festen Phase gemäß Schritt iv. oder v) ein Resuspendieren und Reimmobilisieren der Partikel des Magnetpartikel-Konjugats im Waschpuffer umfasst.
41. Das Verfahren gemäß einer der Ausführungsformen 13 bis 40, wobei das Vorbereitungsreagenz eine wässrige Lösung umfassend HNO₃ und H₂O₂ ist.
42. Das Verfahren gemäß einer der Ausführungsformen 13 bis 41, wobei das Initiationsreagenz eine wässrige Lösung umfassend NaOH und ein Detergens ist.
43. Das Verfahren gemäß einer der Ausführungsformen 13 bis 42, wobei das Auslösen der Chemilumineszenz umfasst:
   a. Zugeben des Vorbereitungsreagenzes;
   b. Durchmischen unter Resuspendieren des Magnetpartikel-Konjugats im Vorbereitungsreagenz; und
   c. Zugeben des Initiationsreagenzes.
44. Das Verfahren gemäß einer der Ausführungsformen 13 bis 43, wobei die Chemilumineszenz mittels Photomultiplier quantifiziert wird.
45. Das Verfahren gemäß einer der Ausführungsformen 13 bis 44, wobei das zu bestimmende freie Thyroid-Hormon fT4 ist.
46. Das Verfahren gemäß einer der Ausführungsformen 13 bis 44 wobei das zu bestimmende freie Thyroid-Hormon fT3 ist.
47. Das Verfahren gemäß einer der Ausführungsformen 13 bis 46, wobei das quantitative Bestimmen des freien Thyroid-Hormons gemäß Schritt h. umfasst:
   (i) Bestimmen der Chemilumineszenz in 2 bis 10 Kalibratorlösungen, wobei die Kalibratorlösungen das zu bestimmende Thyroid-Hormon in unterschiedlichen Konzentrationen und mindestens ein T4-bindendes Protein enthalten, und wobei den Kalibratorlösungen eine Konzentration des zu bestimmenden freien Thyroid-Hormons zugeordnet ist;
   (ii) Erstellen einer Kalibrationskurve anhand der zugeordneten freien Thyroid-Hormon-Konzentrationen der Kalibratorlösungen und der bestimmten Chemilumineszenz; und
   (iii) Quantifizieren des Thyroid-Hormons mittels Vergleich der gemäß Schritt g. gemessenen Chemilumineszenz einer Blutprobe und der unter (ii) erstellten Kalibrationskurve.
48. Kit für einen heterogenen Immunoassay zur Bestimmung eines freien Thyroid-Hormons umfassend
   i. ein Fängerreagenz umfassend ein Magnetpartikel-Konjugat, wobei das Magnetpartikel-Konjugat einen Magnetpartikel und einen spezifischen Binder des entsprechenden Thyroid-Hormones umfasst; und
   ii. ein Detektorreagenz umfassend einen Thyronin-Derivat-Tracer der Formel (II)

      CL-Y-TX (II)

      wobei
      CL für eine Chemilumineszenzgruppe steht,
      Y für ein lineares Peptid bestehend aus 3-12 Aminosäuren steht, wobei mindestens die Hälfte der Aminosäuren des linearen Peptids saure Aminosäuren sind, und
      TX für eine Gruppe der Formel (III) oder (IV) 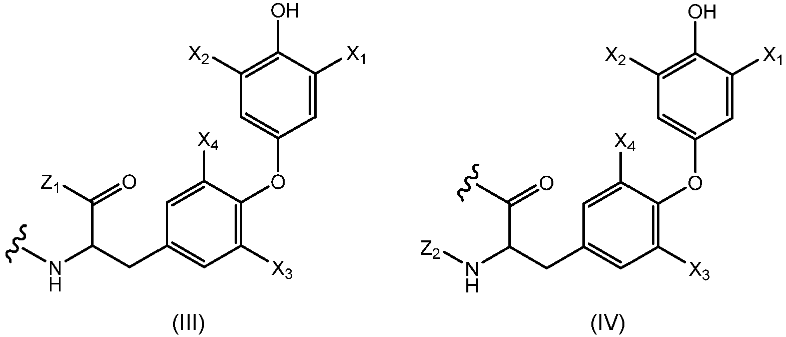 steht,
         wobei X_{1,} X₂, X₃ und X₄ unabhängig voneinander für I, H, Br, CI oder CN stehen,
         Z₁ für OH oder NH₂ steht und
         Z₂ für H oder C(O)CH₃ steht.
49. Das Kit gemäß Ausführungsform 48, ferner umfassend
   iii. einen Waschpuffer, wobei der Waschpuffer ein Detergens umfasst.
50. Das Kit gemäß Ausführungsform 48 oder 49, wobei der Thyronin-Derivat-Tracer ein Thyronin-Derivat-Tracer gemäß einer der Ausführungsformen 1 bis 12 ist.
51. Das Kit gemäß einer der Ausführungsformen 48 bis 50, wobei das Detektorreagenz ein 2-Komponenten-System ist.
52. Das Kit gemäß einer der Ausführungsformen 48 bis 51, wobei X_{1,} X₂, X₃ und X₄ unabhängig voneinander für I oder H stehen.
53. Das Kit gemäß einer der Ausführungsformen 48 bis 52, wobei mindestens eines von X_{1,} X₂, X₃ und X₄ für I steht, bevorzugt mindestens zwei von X_{1,} X₂, X₃ und X₄ für I stehen.
54. Das Kit gemäß einer der Ausführungsformen 48 bis 53, ferner umfassend
   iv. mindestens eine Kalibrationskomponente enthaltend das zu bestimmende Thyroid-Hormon, wobei der Kalibrationskomponente eine freie Thyroid-Hormonkonzentration des zu bestimmenden Hormons zugeordnet ist.

### Beispiele

Die Beispiele zeigen die Durchführung eines erfindungsgemäßen Verfahrens zur quantitativen Bestimmung von fT4. Bei diesem Assay handelt es sich um einen quantitativen, heterogenen, kompetitiven Immunoassay, der als Zweischritt-Assay ausgeführt wird.

Die Chemilumineszenzmessung wurde auf dem Multimode Mikroplattenreader Mithras LB 943 durchgeführt.

Alle Immunoassay-Untersuchungen wurden auf einem vollautomatisierten Immunoassay-Laboranalyzer durchgeführt. Alle Assayschritte und Inkubationszeiten wurden, sofern nicht anders angegeben, bei 37°C durchgeführt.

### Herstellung von Fängerreagenz und Magnetpartikel-Konjugat

Als Antikörper wurde in den Beispielen ausschließlich der von Meridian Bioscience vertriebene anti-T4-Antikörper MAT02-525 (Clone 204-14525) eingesetzt, und mittels Sulfo ChromaLink Biotin (SCLB) (Catalog# B-1007), wie beispielsweise durch die Firma Vector Laboratories vertrieben, kovalent über einen PEG-Linker mit enthaltenem UV-Farbstoff an Biotin gekoppelt. Hierzu wurde der Antikörper mit PBS auf eine Konzentration von 2 mg/mL verdünnt und Sulfo ChromaLink Biotin in wasserfreiem DMSO in 26-fachem molaren Überschuss zugegeben und die Mischung unter Lichtauschluss für 1 h inkubiert. Als Abstopplösung wurde, bezogen auf SCLB, ein 1000facher molarer Überschuss an Glycin zugegeben und das Antikörper-SCLB-Konjugat anschließend über eine Gelfiltrationssäule (Typ HiTrap Desalting, 5 ml, GE Healthcare Bio-Sciences AB) aufgereinigt. Als Chromatographie-System wurde ein Äkta Purifier 10 von Amersham Pharmacia Biotech verwendet.

Als Magnetpartikel wurden in den Beispielen ausschließlich Dynabeads M-270 Streptavidin von Invitrogen eingesetzt. Die Magnetpartikel haben eine Partikelgröße von 2,8 µm (t < 5 %) und sind auf der Oberfläche mit Carbonsäuren funktionalisiert, an die kovalent Streptavidin gebunden ist.

Die Magnetpartikel wurden 2malig mit PBS-Puffer gewaschen und anschließend in PBS-Puffer mit dem Antikörper-SCLB-Konjugat für 1,5 h bei RT inkubiert und geschüttelt. Die Konzentration der Magnetpartikel sowie des Antikörper-SCLB-Konjugates entsprach bei diesem Ankoppelungsschritt der späteren Endkonzentration im Reagenzrezept.

Soweit die Absättigung der Beads mit Biotin im Beispiel vermerkt ist, wurde im Anschluss an den Kopplungsschritt dem Inkubationspuffer Biotin in einer Endkonzentration von 0,3 µg/mL pro enthaltener Beadkonzentration von 1 mg/mL zugegeben (z.B. 0,075 µg/mL Biotin für eine Beadkonzentration von 0,25 mg/mL) und erneut 1,5 h bei RT geschüttelt. Im Anschluss wurde das Magnetpartikelkonjugat dreimal mit PBS-Puffer gewaschen und anschließend im Fängerreagenz-Puffer aufgenommen. Das fertige Fängerreagenz wurde homogenisiert und in die für den Immunoassay-Laboranalyzer vorgesehenen Reagenzkontainer gefüllt.

### Herstellung von Detektor-Reagenz und Thyronin-Derivat-Tracer

Alle peptidischen Thyronin-Derivat-Tracer wurden durch Festphasensynthese durch die Firma Biosynth (Lelystad, Niederlande) hergestellt und mittels UPLC-MS und UPLC-UV charakterisiert. Tabelle 1 zeigt die in den Beispielen eingesetzten Thyronin-Derivat-Tracer inklusive ihrer Tracer-Kurzformel und ihrer Reinheit.

Zur Herstellung des Vergleichstracers T3-BSA-NSPSA wurde T3-BSA der Firma Biospacific (Katalog-Nr. 19000) durch Zugabe von NSPSA-NHS (3-[9-(((3-(N-succinimidyloxycarboxypropyl)[4-Methylphenyl]sulfonyl)amin)carboxyl]-10-acridiniumyl)-1-propansulfonat; CAS-No 199293-83-9) kovalent an den Chemilumineszenzfarbstoff NSPSA gekoppelt. Hierzu wurde T3-BSA mit PBS auf eine Konzentration von 1,85 mg/mL verdünnt und NSPSA-NHS in wasserfreiem DMF in 20-fachem molaren Überschuss zugegeben und die Mischung unter Lichtauschluß für 25 min inkubiert. Das Produkt wurde anschließend über eine Gelfiltrationssäule aufgereinigt (Typ HiTrap Desalting, GE Healthcare Bio-Sciences AB). Als Chromatographie-System wurde ein Äkta Purifier 10 von Amersham Pharmacia Biotech verwendet. Durch Auswertung der Peakflächen des Chromatogramms für die Detektion bei 370 nm konnte für das T3-BSA-NSPSA-Konjugat ein molares Verhältnis von NSPSA-Farbstoff zu BSA von 6,1 ermittelt werden.

Der für das jeweilige Reagenz vorgesehene Tracer wurde mit dem angegebenen Detektorreagenzpuffer auf die vorgesehene Konzentration verdünnt.

Das fertige Detektorreagenz wurde in die für den Immunoassay-Laboranalyzer vorgesehenen Reagenzkontainer gefüllt.

**Tabelle 1: Thyronin-Derivat-Tracer**

| Beispiel | Tracer-Name | Tracer-Kurzformel | Reinheit |
|---|---|---|---|
| CT-A | T3-BSA-NSPSA | [BSA mit angekoppeltem T3 und NSPSA] | - |
| CT-B | T4-Peptid-10 | NSPSA-KKKKK-(IIIe)OH | 98,6 % |
| CT-C | T3-Peptid-5 | NSPSA-KKK-(IIIc)OH | 95,2 % |
| T-A | T4-Peptid-7 | NSPSA-EEEEE-(IIIe)OH | 96,2 % |
| T-B | T3-Peptid-3 | NSPSA-EEEEEEE-(IIIc)OH | 95,7 % |
| T-C | T3-Peptid-1 | NSPSA-EKEKEKEKEKE-(IIIc)OH | 86,5 % |
| T-D | T3-Peptid-4 | NSPSA-EEEEEEEEK-(IVc)H | 92,6 % |
| T-E | T3-Peptid-7 | NSPSA-eeeeeee-(IIIc)-NH₂ | 98,8 % |

| | | | |
|---|---|---|---|
| CT- = Vergleichstracer T- = Tracer gemäß der Erfindung | | | |

### Beispiel 1: T4-basierte Thyronin-Derivat-Tracer

Der Immunoassay erfolgte erfindungsgemäß in zwei Schritten.

Im ersten Assay-Schritt wurden 50 µL Probe zu 50 µL Fängerreagenz gegeben.

Das Fängerreagenz bestand aus 0,25 mg/mL Magnetpatikeln (Dynabeads M270-Streptavidin von invitrogen) gekoppelt an 1 µg/ml MAT02-525-SCLB-Konjugat in 50 mM PBS-Puffer mit 1 % rekombinantem HSA (SIGMAA9731), 0,1 % Maus IgG (von Meridian Bioscience) und 0,05 % NaN₃ bei pH 7,4.

Die Mischung wurde für 18 min bei 37°C inkubiert, magnetisch durch Anlegen eines Magnetfeldes an die Wand des Reaktionsgefäßes separiert und 3-mal mit Waschpuffer gewaschen.

Im zweiten Assay-Schritt wurden die Magnetpartikel von der Wand des Reaktionsgefäßes durch Entfernen des Magnetfelds remobilisiert, mit 50 µL Detektorreagenz (44 ng/mL Thyronin-Derivat-Tracer gemäß Tabelle 2 bzw. 3600 ng/mL T3-BSA-NSPSA jeweils in 50 mM Tris-Puffer, 0,15 M NaCl, 0,3 % Tween 20, 0,05 % NaN₃, pH 8,0) aufgewirbelt und für 4 min bei 37°C inkubiert.

Die Magnetpartikel wurden erneut magnetisch durch Anlegen eines Magnetfeldes an die Wand des Reaktionsgefäßes separiert und erneut 3-mal mit Waschpuffer gewaschen.

100 µL Vorbereitungsreagenz-Lösung (0,075 % HNO₃, 1,32 % H₂O₂, 0,027 % Triton X-100) und anschließend 300 µL Initiationsreagenz-Lösung (1,39 % NaOH, 2 % Triton X-100) wurden zugegeben, um das Chemilumineszenz-Signal auszulösen. Das Chemilumineszenz-Signal wurde mit dem Photomultiplier aufgenommen.

Die Ergebnisse sind in Tabelle 2 dargestellt. Die Ergebnisse für die Bestimmung der Chemilumineszenz nach Verdünnung um Faktor 15 Millionen (direkte Zugabe von Pretrigger und Trigger zur verdünnten Lösung ohne Durchführung eines Immunoassays) sind hierbei prozentual auf die Ergebnisse der Referenz CT-A bezogen und pro enthaltener NSPSA-Konzentration dargestellt.

Der LOD-Wert (Limit of Detection) beschreibt die Sensitivität des Immunoassays. Er entspricht der doppelten Standardabweichung berechnet aus einer 10fach-Bestimmung der Nullmatrixmessung geteilt durch den Betrag der Steigung der Kalibrierfunktion im unteren Konzentrationsbereich.

Zur Bestimmung der unspezifischen Anbindung an den Magnetpartikel wurde ein zweites Fängerreagenz ,F-0' auf gleiche Weise hergestellt, dem jedoch kein Antikörper-SCLB-Konjugat zugegeben wurde. Da dieses Fängerreagenz F-0 kein spezifisches Anbindungselement für den Tracer enthält, kann der Tracer in Kombination mit diesem Fängerreagenz nur unspezifisch an die Beads binden. Zur Berechnung des unspezifischen Anbindungsanteils des Assays wurde daher das Signal der Assays mit Fängerreagenz F-0 zum Assaysignal mit dem normalen Fängerreagenz prozentual ins Verhältnis gesetzt, wobei als Probe ein euthyreoder Serumpool verwendet wurde.

**Tabelle 2: Performance T4-basierter Thyronin-Derivat-Tracer im Vergleich zum Vergleichstracer T3-BSA-NSPSA (CT-A)**

| Beispiel | Tracer-Kurzformel | Chemilumineszenz nach Verdünnung um Faktor 15 Mio ¹ | Sensitivität LOD [pmol/L] | Unspezifische Anbindung an Magnetpartikel |
|---|---|---|---|---|
| CT-A | [BSA mit angekoppeltem T3 und NSPSA] | Referenz | 2,0 | 6,40 % |
| CT-B | NSPSA-KKKKK-(IIIe)OH | 19 % | 13,7 | 3,90 % |
| T-A | NSPSA-EEEEE-(IIIe)OH | 72 % | 13,2 | 1,20 % |

| | | | | |
|---|---|---|---|---|
| ¹ Verdünnungsmatrix: 10 mM PBS + 0,05 % Tween 20 + 0,5 % BSA, pH 7,4 | | | | |

Es wird gezeigt, dass im Vergleich zum handelsüblichen Tracer (T3-BSA-NSPSA) die Chemilumineszenzstärke und die Sensitivität des Assays geringer ist, eine unspezifische Anbindung an die Magnetpartikel jedoch drastisch reduziert werden kann (siehe Beispiel CT-Aggü. Beispiel T-A). Es ist anzunehmen, dass der Austausch des Makromoleküls BSA (Molmasse von BSA etwa 66.000 Da) durch den niedermolekularen, stark negativ geladenen Linker von T-A (Molmasse T-A etwa 1.990 Da) für die stark reduzierte unspezifische Anbindung im Assay von CT-A nach T-A verantwortlich ist. Es muss allerdings davon ausgegangen werden, dass die Bindungsrivalität gegenüber dem anti-T4-Antikörper bei T4-basierten Tracern (T-A) höher als bei T3-basierten Tracern (CT-A) ist, was die geringere Sensitivität in Beispiel T-A erklärt.

Es wird gezeigt, dass bei vergleichbaren Sensitivitäten die Chemilumineszenzstärke deutlich geringer ist und die unspezifische Anbindung an die Magnetpartikel höher ist, wenn ein kurzer basischer Peptid-Linker (CT-B) anstelle eines kurzen sauren Peptid-Linkers (T-A) verwendet wird.

### Beispiel 2: T3-basierte Thyronin-Derivat-Tracer

Der Immunoassay erfolgte erfindungsgemäß in zwei Schritten.

Im ersten Assay-Schritt wurden 50 µL Probe zu 50 µL Fängerreagenz gegeben.

Das Fängerreagenz bestand aus 0,25 mg/mL Magnetpatikeln (Dynabeads M270-Streptavidin von invitrogen) gekoppelt an 1 µg/ml MAT02-525-SCLB-Konjugat in 100 mM PBS-Puffer mit 1 % rekombinantem HSA (SIGMAA9731), 0,1 % Maus IgG (von Meridian) und 0,05 % NaN₃ bei pH 7,0.

Die Mischung wurde für 18 min bei 37°C inkubiert, magnetisch durch Anlegen eines Magnetfeldes an die Wand des Reaktionsgefäßes separiert und 3 mal mit Waschpuffer gewaschen.

Im zweiten Assay-Schritt wurden die Magnetpartikel von der Wand des Reaktionsgefäßes durch Entfernen des Magnetfelds remobilisiert, mit 50 µL Detektorreagenz (40 ng/mL Thyronin-Derivat-Tracer gemäß Tabelle 3 bzw. 1200 ng/mL T3-BSA-NSPSA in 60 mM Barbital-Puffer mit 0,15 M NaCl, 1 % rekombinantem HSA (SIGMAA9731), 0,1 % Maus IgG (von Meridian), 0,05 % NaN₃, pH 8,6) aufgewirbelt und für 4 min bei 37°C inkubiert.

Die Magnetpartikel wurden magnetisch durch Anlegen eines Magnetfeldes an die Wand des Reaktionsgefäßes separiert und erneut 3 mal mit Waschpuffer gewaschen.

100 µL Lösung des Vorbereitungsreagenzes (0,027 % HNO₃, 1,32 % H₂O₂, 0,027 % Triton X-100) und anschließend 300 µL Lösung des Initiationsreagenzes (1,39 % NaOH, 2 % Triton X-100) wurden zugegeben, um das Chemilumineszenz-Signal auszulösen. Das Chemilumineszenz-Signal wurde mit dem Photomultiplier aufgenommen.

Die Ergebnisse sind in Tabelle 3 dargestellt. Zur Bestimmung des Korrelationskoeffizienten r² wurden 60 euthyreode Serumproben von potentiell gesunden, humanen Einzelspendern sowohl mit den hier beschriebenen Immunoassays als auch mit dem Cobas fT4 Assay als Referenzsystem bestimmt. Die Ergebnisse eines jeden der hier beschriebenenen Assays wurde mit den Ergebnissen des Cobas fT4-Assays nach least-squares fit korreliert und der Korrelationskoeffizient r² errechnet.

Der Offset (auch Offset an der oberen Messbereichsgrenze (OMG)) ist der Quotient des Signals des Kalibrators mit der höchsten fT4-Konzentration und des Signals des Null-Kalibrators. Ein hoher Offset (insbesondere >30 %) kann einen Hinweis darauf sein, dass der Tracer, der im Assayschritt 2 zugegeben wird in signifikanten Maße Antigen von den Antikörperbindungsstellen verdrängt, was sich negativ auf die Assayperformance auswirken kann.

**Tabelle 3: Performance T3-basierter Thyronin-Derivat-Tracer**

| Beispiel | Tracer-Kurzformel | Korrelationskoeffizient r2 | Sensitivität LOD [pmol/L] | Offset |
|---|---|---|---|---|
| CT-A | [BSA mit angekoppeltem T3 und NSPSA] | 0,75 | 1,8 | 27 % |
| CT-C | NSPSA-KKK-(IIIc)OH | 0,42 | 4 | 44 % |
| T-B | NSPSA-EEEEEEE-(IIIc)OH | 0,72 | 1,1 | 10 % |
| T-C | NSPSA-EKEKEKEKEKE-(IIIc)OH | 0,53 | 2,1 | 5 % |
| T-D | NSPSA-EEEEEEEEK-(IVc)H | 0,64 | 1,5 | 6 % |

Die Ergebnisse zeigen einen geringen prozentualen Offset bei Verwendung niedermolekularer T3-basierter Tracer gemäß der vorliegenden Erfindung, der auch im Vergleich zu kommerziell erhältlichen T3-Albumin-Konjugaten deutlich niedriger ist. Zudem kann bei Verwendung saurer Peptid-Linker (Beispiel T-B) eine höhere Sensitivität erzielt werden. Während gemischte Linker (mit sauren und basischen Aminosäuren, Beispiel T-C) zwar wünschenswert niedrige Werte für den Offset zeigen, fallen die Sensitivität und der Korrelationskoeffizient r² schlechter aus als bei Verwendung saurer Peptid-Linker. Die Verwendung basischer Linker zeigt keine zufriedenstellenden Ergebnisse.

Wird unter Verwendung eines sauren Linkers die T3-Gruppe über ihre Carboxy-Gruppe an das Peptid gebunden (Beispiel T-D), so werden Ergebnisse mit geringfügig schlechterer Sensitivität und Korrelationskoeffizienten beobachtet. Es wird jedoch vermutet, dass es vom verwendeten Antikörper abhängt, ob die Anbindung der TX-Gruppe über die AminoGruppe oder die Carboxy-Gruppe besser funktioniert.

### Beispiel 3: Einfluss von rHSA

Der Assay wird analog zu Beispiel 2 durchgeführt. Als Detektorpuffer wurde 50 mM PBS mit 0,1 % Maus IgG und 0,05 % NaN₃ und rHSA Varianten-abhängig nach Tabelle 4 bei pH 7,7 verwendet. Die Tracerkonzentration im Detektorreagenz betrug 15 ng/mL. Der Einfluss unterschiedlicher Mengen rHSA im Detektorreagenz wurde ermittelt.

Die Ergebnisse sind in Tabelle 4 dargestellt. Der LOQ (Limit of Quantitation) beschreibt, ab welcher Konzentration der Test eine Probe mit mindestens einer angegebenen Präzision (hier ≤10 %) bestimmen kann und wurde ermittelt, indem 5 Proben mit fT4-Konzentrationen im Bereich 3-10 pmol/L in 10fach-Bestimmung gemessen wurden. Es wurde der Variationskoeffizient der Konzentrationbestimmungen für jede Probe errechnet und durch Regression ermittelt, bei welcher fT4-Konzentration der Variationskoeffizient im Mittel bei 10 % lag.

**Tabelle 4: Einfluss von rHSA im Detektorreaenz**

| Tracer | Tracer-Kurzformel | Konzentration von rHSA im Detektorreagenz | Korrelationskoeffizient r² | Sensitivität LOQ [pmol/L] |
|---|---|---|---|---|
| T-B | NSPSA-EEEEEEE-(IIIc)OH | 1 % | 0,83 | 4,5 |
| T-B | NSPSA-EEEEEEE-(IIIc)OH | kein rHSA | 0,74 | 8,0 |

Die Anwesenheit von 1 % rHSA im Detektorreagenz führt zu einer deutlichen PerformanceVerbesserung im Falle des LOQ sowie zu einer leichten Performanceverbesserung im Falle der Korrelation zum Referenzsystem.

### Beispiel 4: Absättigung der Beads mit Biotin

Der Assay wird analog zu Beispiel 2 durchgeführt.

Das Fängerreagenz bestand aus 0,25 mg/mL Magnetpatikeln (Dynabeads M270-Streptavidin von invitrogen) gekoppelt an 1 µg/mL MAT02-525-SCLB-Konjugat in 100 mM PBS-Puffer mit 1 % rekombinantem HSA (SIGMAA9731), 0,1 % Maus IgG (von Meridian) und 0,05 % NaN₃ bei pH 7,0. Für eine Reagenzvariante (siehe Tabelle 5) wurden die Beads, wie oben beschrieben, nach der Ankoppelung des MAT02-525-SCLB-Konjugates mit 0,075 µg/mL Biotin abgesättigt (weitere 1,5 h Inkubationszeit). Für die andere Reagenzvariante wurde dieser Absättigungsschritt nicht durchgeführt. Die Menge des für die Absättigung der Beads verwendeten Biotins entsprach in etwa 90 % der nach der Kopplung des Antikörper-SCLB-Konjugates noch freien Biotin-Bindungsplätze der Beads M270-Streptavidin.

Als Detektorpuffer wurde 50 mM PBS mit 1 % rHSA und 0,05 % NaN3 bei pH 7,5 verwendet. Die Tracerkonzentration von T-B im Detektorreagenz betrug 25 ng/mL.

Die Ergebnisse sind in Tabelle 5 dargestellt. Zur Bestimmung der Biotininterferenz des Assays wurde zu einer humanen Serumpool-Probe 1200 ng/mL Biotin gegeben (Zugabe einer Stammlösung von 0,2 mg/mL Biotin in Reinwasser). Als Referenzprobe diente die Mischung der gleichen Serumpool-Probe und Reinwasser im gleichen VolumenVerhältnis.

Für jede der Assay-Varianten wurde sowohl die Biotin-haltige Probe als auf die Referenzprobe in siebenfach-Bestimmung alternierend gemessen. Es wurde berechnet, um wieviel Prozent die mittlere bestimmte fT4-Konzentration der Biotin-haltigen Probe von der Referenzprobe abwich.

**Tabelle 5: Einfluss der Biotin-Absättigung des Magnetpartikel-Konjugats auf die Biotin-Interferenz des Assays**

| Tracer | Tracer-Kurzformel | Absättigung des Magnetpartikelkonjugats mit Biotin | Biotin-Interferenz des Assays (1200 ng/mL Biotin) |
|---|---|---|---|
| T-B | NSPSA-EEEEEEE-(IIIc)OH | Keine Absättigung | 51,5 % |
| T-B | NSPSA-EEEEEEE-(IIIc)OH | Absättigung mit 75 ng/mL Biotin | 2,2 % |

Die Ergebnisse zeigen, dass sich die deutliche Biotin-Interferenz des Assays durch Absättigung der Beads mit Biotin fast komplett beheben lässt.

### Beispiel 5: Tracer mit D-Aminosäure-Linker und Z₁=NH₂

Der Assay wird analog zu Beispiel 2 durchgeführt.

Als Detektorreagenzpuffer wurde 50 mM PBS mit 1 % rHSA, 0,02 % 4-Methoxyphenol, 0,5 mM EDTA und 0,05 % NaN₃ bei pH 7,3 eingesetzt. Als Tracer werden entweder 15 ng/mL T-B oder 25 ng/mL T-E verwendet.

Das Fängerreagenz enthielt 0,44 mg/mL M270-Streptavidin-Beads gekoppelt an 1,75 µg/mL MAT02-525-SCLB-Konjugat und wurde mit Biotin abgesättigt, wie oben beschrieben. Der Fängerreagenzpuffer bestand aus 100 mM PBS, 1 % rHSA, 0,1 % Maus IgG und 0,05 % NaN3 bei pH 7,0.

Die Ergebnisse sind in Tabelle 6 dargestellt. Zur Bestimmung der Kalibrationsstabilität wurden die zu testenden Reagenzien für 43 Tage 24 h am Tag auf dem Immunoassay-Analyzer gelagert, wobei sie ständig geschüttelt und bei 4-8 °C gelagert wurden. An Tag 0 wurden die Reagenzien kalibriert. An Tag 0, 4, 11, 21, 31 und 43 wurde jeweils ein Aliquot einer fT4-Probe mit ca. 8 pmol/L fT4 und eine Probe mit ca. 16 pmol/L fΓ4 aufgetaut (Lagerung bei -20°C) und jeweils in vierfach Bestimmung gemessen. Die Änderung der Probenkonzentration während des Messzeitraumes wurde über least squares fit gemittelt und auf einen Zeitraum von 10 Tagen heruntergerechnet.

**Tabelle 6: Einfluss des Tracer-Typs auf die Kalibrationsstabilität**

| | | Kalibrations-Stabilität Gemittelte fT4-Konzentrations-Zunahme in 10 d | |
|---|---|---|---|
| Tracer im Detektorreagenz | Tracer-Kurzformel | Niedrige Probe | Hohe Probe |
| 15 ng/mL T-B | NSPSA-EEEEEEE-(IIIc)OH | 4,0% | 3,5% |
| 25 ng/mL T-E | NSPSA-eeeeeee-(IIIc)-NH₂ | 0,3% | 0,6% |

Es ist ersichtlich, dass die gemessene Probenkonzentration bei Verwendung von Tracer T-E im Messzeitraum stabiler bleiben und Tracer T-E damit eine höhere Kalibrationsstabilität besitzt als der Tracer T-B.

## Patentansprüche

1. Thyronin-Derivat-Tracer der Formel (II)
CL-Y-TX (II)
wobei
CL für eine Chemilumineszenzgruppe steht,
Y für ein lineares Peptid bestehend aus 3 bis 12 Aminosäuren steht, wobei mindestens die Hälfte der Aminosäuren des linearen Peptids saure Aminosäuren sind, und
TX für eine Gruppe der Formel (III) oder (IV) 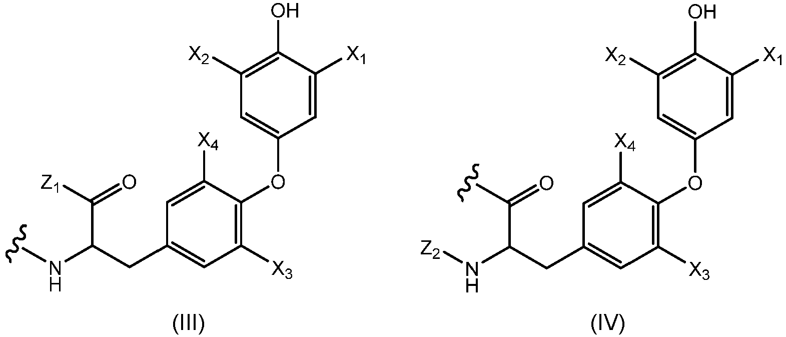
steht,
wobei X_{1,} X₂, X₃ und X₄ unabhängig voneinander für I, H, Br, CI oder CN stehen, Z₁ für OH oder NH₂ steht und
Z₂ für H oder C(O)CH₃ steht.

2. Der Thyronin-Derivat-Tracer gemäß Anspruch 1, wobei die Aminosäuren D-Aminosäuren sind.

3. Der Thyronin-Derivat-Tracer gemäß einem der vorstehenden Ansprüche, wobei X_{1,} X₃ und X₄ für I stehen und X₂ für H steht.

4. Der Thyronin-Derivat-Tracer gemäß einem der vorstehenden Ansprüche, wobei Z₁ für NH₂ steht.

5. Verfahren zur quantitativen Bestimmung eines freien Thyroid-Hormons umfassend
a. Bereitstellen einer Blutprobe;
b. Kombinieren der Blutprobe mit einem Fängerreagenz in einem Reaktionsgefäß unter Erhalt eines ersten Gemisches,
wobei das Fängerreagenz ein Magnetpartikel-Konjugat umfasst, und
wobei das Magnetpartikel-Konjugat einen Magnetpartikel und einen spezifischen Binder des zu bestimmenden Thyroid-Hormons umfasst;
c. Aufarbeiten des ersten Gemisches zumindest umfassend
i. Inkubieren des ersten Gemisches;
ii. Immobilisieren des Magnetpartikel-Konjugats an der Wand des Reaktionsgefäßes;
iii. Entfernen der flüssigen Phase; und
iv. Waschen der festen Phase durch mehrmalige Abgabe und Aufnahme eines Waschpuffers;
d. Zugeben eines Detektorreagenzes in das Reaktionsgefäß unter Erhalt eines zweiten Gemisches,
wobei das Detektorreagenz einen Thyronin-Derivat-Tracer der Formel (II)
CL-Y-TX (II)
umfasst, wobei
CL für eine Chemilumineszenzgruppe steht,
Y für ein lineares Peptid bestehend aus 3 bis 12 Aminosäuren steht, wobei mindestens die Hälfte der Aminosäuren des linearen Peptids saure Aminosäuren sind, und
TX für eine Gruppe der Formel (III) oder (IV) 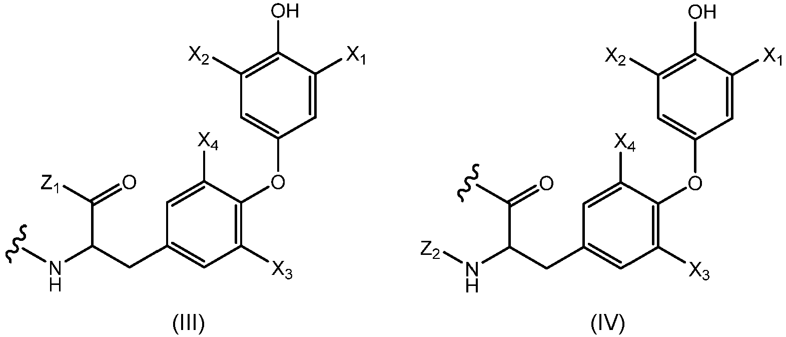
steht,
wobei X_{1,} X₂, X₃ und X₄ unabhängig voneinander für I, H, Br, CI oder CN stehen,
Z₁ für OH oder NH₂ steht und
Z₂ für H oder C(O)CH₃ steht;
e. Aufarbeiten des zweiten Gemisches zumindest umfassend
i) Resuspendieren der Partikel des Magnetpartikel-Konjugats im Detektorreagenz;
ii) Inkubieren des zweiten Gemisches;
iii) Immobilisieren des Magnetpartikel-Konjugats an der Wand des Reaktionsgefäßes;
iv) Entfernen der flüssigen Phase; und
v) Waschen der festen Phase durch mehrmalige Abgabe und Aufnahme eines Waschpuffers;
f. Auslösen der Chemilumineszenz des an das Magnetpartikel-Konjugat gebundenen Thyroid-Derivat-Tracers durch Zugabe mindestens eines Vorbereitungsreagenzes und mindestens eines Initiationsreagenzes;
g. Messen der Chemilumineszenz; und
h. quantitatives Bestimmen des freien Thyroid-Hormons.

6. Das Verfahren gemäß Anspruch 5, wobei das Detektorreagenz ein Albumin, optional in einer Menge von 0,1 bis 5 %, bezogen auf die Gesamtmasse Detektorreagenz, umfasst und/oder wobei das Fängerreagenz ein Albumin, optional in einer Menge von 0,1 bis 5 %, bezogen auf die Gesamtmasse Fängerreagenz, umfasst.

7. Das Verfahren gemäß Anspruch 6, wobei das im Detektorreagenz, im Fängerreagenz oder im Detektor- und Fängerreagenz enthaltene Albumin rekombinantes Albumin oder mit Aktivkohle aufgereinigtes Albumin ist.

8. Das Verfahren gemäß einem der Ansprüche 5 bis 7, wobei das Detektorreagenz als 2-Komponenten-System bereitgestellt wird.

9. Das Verfahren gemäß einem der Ansprüche 5 bis 8, wobei der Thyronin-Derivat-Tracer im Detektorreagenz in einer Konzentration von 0,1 bis 400 ng/mL enthalten ist.

10. Das Verfahren gemäß einem der Ansprüche 5 bis 9, wobei das zu bestimmende freie Thyroid-Hormon fT4 oder fT3 ist.

11. Das Verfahren gemäß einem der Ansprüche 5 bis 10, wobei das quantitative Bestimmen des freien Thyroid-Hormons gemäß Schritt h. umfasst:
(i) Bestimmen der Chemilumineszenz in 2 bis 10 Kalibratorlösungen,
wobei die Kalibratorlösungen das zu bestimmende Thyroid-Hormon in unterschiedlichen Konzentrationen und mindestens ein T4-bindendes Protein enthalten, und wobei den Kalibratorlösungen eine Konzentration des zu bestimmenden freien Thyroid-Hormons zugeordnet ist;
(ii) Erstellen einer Kalibrationskurve anhand der zugeordneten freien Thyroid-Hormon-Konzentrationen der Kalibratorlösungen und der bestimmten Chemilumineszenz; und
(iii) Quantifizieren des Thyroid-Hormons mittels Vergleich der gemäß Schritt g. gemessenen Chemilumineszenz einer Blutprobe und der unter (ii) erstellten Kalibrationskurve.

12. Kit für einen heterogenen Immunoassay zur Bestimmung eines freien Thyroid-Hormons umfassend
i. ein Fängerreagenz umfassend ein Magnetpartikel-Konjugat, wobei das Magnetpartikel-Konjugat einen Magnetpartikel und einen spezifischen Binder des entsprechenden Thyroid-Hormones umfasst; und
ii. ein Detektorreagenz umfassend einen Thyronin-Derivat-Tracer der Formel (II)
CL-Y-TX (II)
wobei
CL für eine Chemilumineszenzgruppe steht,
Y für ein lineares Peptid bestehend aus 3-12 Aminosäuren steht, wobei mindestens die Hälfte der Aminosäuren des linearen Peptids saure Aminosäuren sind, und
TX für eine Gruppe der Formel (III) oder (IV) 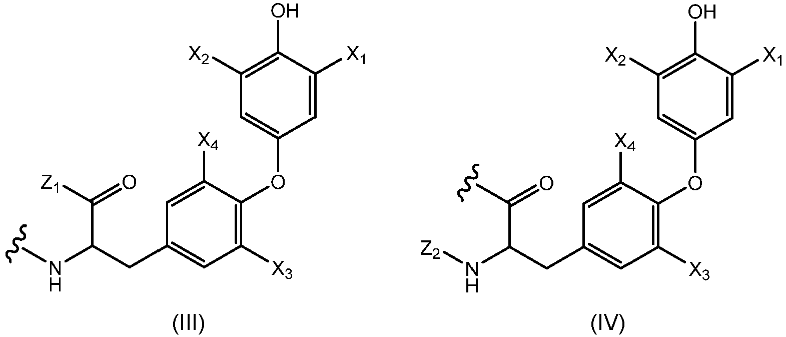
steht,
wobei X_{1,} X₂, X₃ und X₄ unabhängig voneinander für I, H, Br, CI oder CN stehen,
Z₁ für OH oder NH₂ steht und
Z₂ für H oder C(O)CH₃ steht.

13. Das Kit gemäß Anspruch 12, ferner umfassend
iii. einen Waschpuffer, wobei der Waschpuffer ein Detergens umfasst.

14. Das Kit gemäß einem der Ansprüche 12 oder 13, wobei das Detektorreagenz ein 2-Komponenten-System ist.

15. Das Kit gemäß einem der Ansprüche 12 bis 14, ferner umfassend
iv. mindestens eine Kalibrationskomponente enthaltend das zu bestimmende Thyroid-Hormon, wobei der Kalibrationskomponente eine freie Thyroid-Hormonkonzentration des zu bestimmenden Hormons zugeordnet ist.
